# EUROPEAN PATENT SPECIFICATION

(11) **EP 2 272 978 B1**
(45) Date of publication and mention of the grant of the patent: **18.04.2012**
(21) Application number: 09165252.9
(22) Date of filing: 10.07.2009
(51) Int. Cl.: C12Q 1/68

(54) **Highly sensitive rapid isothermal method for the detection of point mutations and SNPs**
Hochsensibles schnelles isothermisches Verfahren zur Detektion von Punktmutationen und SNPs
Procédé isothermique rapide et hautement sensible pour la détection de mutations ponctuelles et SNP

(43) Date of publication of application: 12.01.2011
(73) Proprietor: Biotrin International Limited, Stillorgan Industrial Park Dublin (IE)
(72) Inventor: Adlerstein, Daniel, 20014 Nerviano (MI) (IT); Amicarelli, Giulia, 20014 Nerviano (MI) (IT); Minnucci, Giulia, 20151 Milano (IT)
(74) Representative: Comoglio, Elena

(56) References cited:
- EP-A2- 1 975 249
- WO-A1-2009/049630
- WO-A2-2007/011901
- US-A1- 2007 218 464
- NAGAMINE K ET AL: "Accelerated reaction by loop-mediated isothermal amplification using loop primers." MOLECULAR AND CELLULAR PROBES JUN 2002, vol. 16, no. 3, June 2002 (2002-06), pages 223-229, XP002573824 ISSN: 0890-8508
- MURUGESAN GURUNATHAN ET AL: "Identification of the JAK2 V617F mutation in chronic myeloproliferative disorders using FRET probes and melting curve analysis." AMERICAN JOURNAL OF CLINICAL PATHOLOGY APR 2006, vol. 125, no. 4, April 2006 (2006-04), pages 625-633, XP002568551 ISSN: 0002-9173

## Description

### Field of the invention

The current invention refers to a method for detecting point mutations of a nucleotide sequence by an improvement of the LAMP (loop amplification mediated polymerization) amplification method. As non limitative embodiment the invention refers to the G1849T mutation of the JAK2 gene.

### Background

Myeloproliferative disorders (MPD) are clonal disorders of haematopoietic progenitors, and include the classical MPD chronic myeloid leukaemia (CML), polycythaemia vera (PV), essential thrombocythaemia (ET) and primary myelofibrosis (PMF), as well as chronic eosinophilic leukaemia (CEL), chronic myelomonocytic leukaemia (CMML), and systemic mastocytosis (SM) and others. In the past two decades, mutant alleles have been identified in CML, CMML, CEL and SM2-5, and in each case the causative mutation results in constitutive activation of tyrosine kinase signalling. The genetic causes of the most common MPD remained unknown until the identification of mutations that activate Janus kinase 2 (JAK2) signalling in most patients with PV, ET or PMF(1, 2, 3, 4). JAK2 is a member of the Janus family of cytoplasmic non-receptor tyrosine kinases, which also includes JAK1, JAK3 and TYK2. The mutation is a guanine-to-thymidine substitution at base 1489 (GenBank accession no. NM_004972), which results in a substitution of valine for phenylalanine at amino acid 617 of the JAK2 protein (JAK2V617F), within the JH2 pseudokinase domain (5). Loss of JAK2 autoinhibition results in constitutive activation of the kinase, analogous to other mutations in MPDs and leukemia that aberrantly activate tyrosine kinases (6,7,8). Direct sequencing is only sensitive down to about 20% of mutant DNA in a wild-type background (9, 10). This issue is quite relevant to chronic myeloid disorders, where blood and marrow are often composed of a mixture of neoplastic and residual normal hematopoietic elements. Especially in the case of ET and MDS, in which phenotypically apparent gene mutations may be present in tiny clones comprising less than 10% of the total marrow cell population. James et al. (11) explored this issue specifically with respect to JAK2 1849 G-T by performing a series of mixing experiments with HEL erythroleukemia cells, which bear the JAK2 mutation, admixed with TF-1 erythroleukemia cells, which do not. They failed to detect the mutated allele when it was present in <5% of the total DNA. With homozygous mutant patient DNA diluted in DNA from a healthy person, sequencing was even less sensitive (10%) than it was with the cell lines (12).

A common method used is the Amplification Refractory Mutation System (ARMS). It exploits the fact that oligonucleotide primers must be perfectly annealed at their 3' ends for a DNA polymerase to extend these primers during PCR (12). By designing oligonucleotide primers that match only a specific DNA point mutation, such as that encoding JAK2 V617F, ARMS can distinguish between polymorphic alleles. Therefore, these techniques go by the alternative names of "allele-specific PCR" (AS-PCR) or "sequence-specific primer PCR." The ARMS sensitivity is up to 1 to 2% (13) mutant DNA in a wild-type background.

Real-time monitoring of PCR product accumulation during thermocycling can be of value as a semiquantitative method and DNA-melting curve assays can be used in conjunction with real-time PCR. Likewise, James et al. (14) compared fluorescent dye chemistry sequencing with two different real-time PCR based mutation detection systems, one using a LightCycler instrument (Roche Diagnostics) and the other using a Taqman ABI Prism 7500 machine (Applied Biosystems). These real-time PCR techniques detected 0.5 to 1% of HEL cell line DNA diluted in TF-1 cell line DNA and 2 to 4% of homozygously mutated patient DNA diluted in DNA from a healthy person.

A Restriction Fragment Length Polymorphism (RFLP) analysis is possible since the JAK2 1849 G-T mutation abolishes a motif in the wild-type *JAK2* sequence that is recognized by the restriction enzyme *Bsa*XI. Although abolition of a restriction site is not as satisfying as creation of a new site, because a negative enzymatic cleavage reaction could be due either to absence of the mutation or to failure of the digestion procedure, it can be useful as a first pass analysis. Reported proportional sensitivity depends in part on the method used to detect the fragments and is approximately 20% mutant DNA in wild-type background (15, 16).

Pyrosequencing is a method of rapid genotyping that depends on the liberation of pyrophosphate (PPi) whenever a dNTP is incorporated into a growing DNA chain during template-driven DNA polymerization (17). Pyrosequencing of *JAK2* using the automated PSQ HS 96 system (Biotage, Uppsala, Sweden) has been attempted by several groups (17, 18) with dilution experiments similar to those described above showing a reported assay sensitivity of 5 to 10% mutant allele in a wild-type background.

Several other mutation detection techniques have been described, including single stranded conformational polymorphism (SSCP) analysis, denaturing gradient gel electrophoresis (DGGE), denaturing high-performance liquid chromatography (DHPLC), single-nucleotide primer extension assays (Pronto), and others. In fact, DHPLC can detect the genomic DNA mutation underlying JAK2 V617F reliably, and it can detect mutations at a proportionality of <1 to 2%. However, DHPLC and the other techniques are either technically challenging or labor-intensive or both. They either do not allow high throughput at a cost suitable for a clinical laboratory (SSCP and DGGE) or require a considerable initial investment for equipment (DHPLC).

Theoretically, protein-based techniques could also be used to detect the JAK2 V617F mutation, but these are generally cumbersome, and access to such resources is limited. Therefore, protein-based assays are usually not preferred if DNA- or RNA-based tests are feasible.

EP1692281 discloses a method of JAK2 mutation detection based on PCR amplification. The method described presents several limitations. First of all the lower level of sensitivity, that allows detection of the mutant sequences of JAK2 down to 1 % of the sample in the best cases. This sensitivity requires the enrichment of the mutants via granulocytes- isolation before extraction. This step is time consuming and labor intensive and add about 2 hours to the already long procedures (from 2 to 5 hours) requested for the diagnosis. Furthermore, all the methods described are relatively labor intensive and expensive, often requiring specialized equipment that may not always be readily available.

Methods for loop-mediated isothermal amplification (LAMP) employing inner primers FIP and BIP and loop primers LB or LF, in addition to outer primers F3 and B3, are disclosed in International patent application W02009/049630; US patent application publication No. US 2007/218464; European patent application EP1975249; as well as in reference NA-GAMINE et al., Clinical Chemistry, Vol. 47, No. 9, September 2001, pages 1742-1743.

### Description of the invention

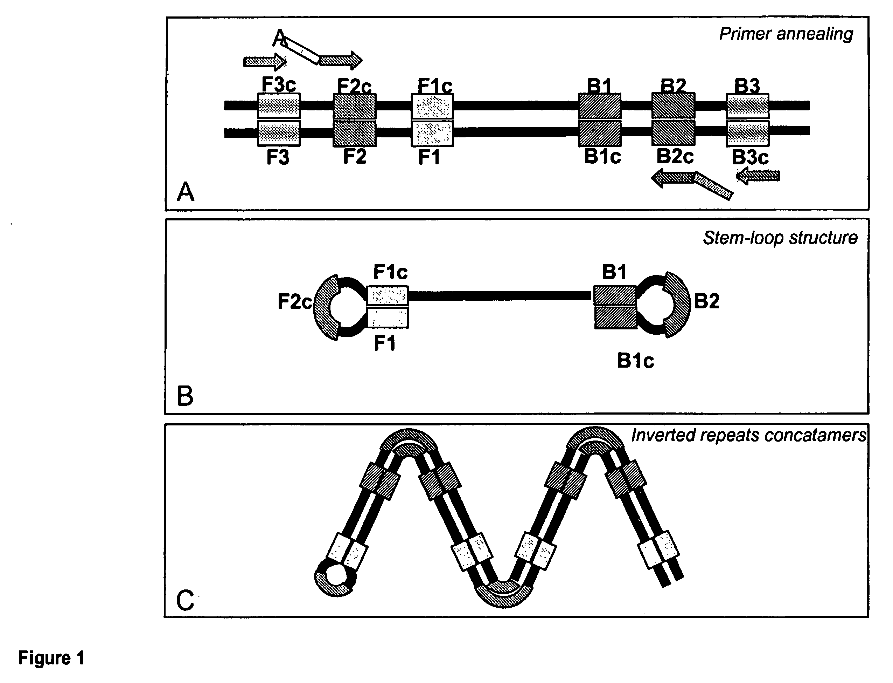

The authors of the current invention have set up a novel method for the detection of point mutations that is selective and rapid. The method departs from the LAMP technology, as disclosed in EP 1020534 and depicted here in Fig. 1.

The assay is easy to be performed, since it needs simple instrumentation and produces the results in a single tube reaction. For these reasons it is also less expensive in respect to the other methods described above.

The method overcomes the limitations underlined in the other techniques. It is more selective (down to 0.01% mutant sequences in wt background); it is isothermal and rapid, completing the diagnosis in a one hour reaction.

The method refers to a simultaneous selective amplification and detection of a single base substitution in nucleic acids.

Therefore it is an object of the invention a method for detecting the presence of a point mutation of a target nucleic acid molecule in a background of nucleic acid wild type molecules, comprising the steps of:
a. obtaining a nucleic acid sample;
b. contacting said nucleic acid sample, in appropriate reaction conditions, with a solution comprising a mixture of oligonucleotides and a DNA polymerase with strand displacement activity under hybridization conditions, wherein said mixture of oligonucleotides consists of primers suitable for a loop mediated isothermal amplification of the region of the nucleic acid molecule putatively including the point mutation, said primers comprising :
   i) two outer primers F3 and B3 ;
   ii) two inner primers FIP and BIP, where FIP includes two oligonucleotide sequences, F2 and F1c, and BIP includes two oligonucleotide sequences, B2 and B1c, where said inner primers FIP and BIP are able to recognize and hybridize to two different and opposite regions, F2c and B2c respectively, of the target nucleic acid molecule, where either the BIP primer is designed to hybridize downstream of the point mutation, or the FIP primer is designed to hybridize upstream of the point mutation, and in the case that the BIP primer is designed to hybridize downstream of the point mutation, then the FIP primer is designed to hybridize to the target sequence such that the point mutation is located in or downstream of the F2c sequence and upstream of F1c, or in the case that the FIP primer is designed to hybridize upstream of the point mutation, then the BIP primer is designed to hybridize to the target sequence such that the point mutation is located in or upstream of the B2c sequence and downstream of B1c;
   iii) a self-annealable extensible primer LB or LF respectively comprising: - a central loop sequence able to selectively recognize and hybridize to the region comprising the putative point mutation of the nucleic acid molecule only if the point mutation is present, - a 5' end sequence and - a 3' end sequence, said 5' end and said 3' end sequences being complementary to each other to form a stem, so that said central loop sequence has an higher hybridization affinity to the region comprising the putative point mutation of the nucleic acid molecule than the hybridization affinity of the 5' end sequence to the 3' end sequence, so that it results in annealing and amplification of the region comprising the putative point mutation of the nucleic acid molecule;
   iv) a non extensible moiety able to selectively recognize and hybridize to the WT sequence of nucleic acid molecule;
c. incubating the resulting mixture at a constant temperature;
d. detecting a signal indicative of amplification of the nucleotide molecule comprising the point mutation.

In a preferred embodiment the point mutation is located in the region between F2 and F1c or B2 and B1c; in an alternative preferred embodiment the point mutation is located in the region probed by B2 or F2.

In a preferred embodiment the sequence at the 5' end and the sequence at the 3' end of said self-annealable extensible primer is of at least 3 nucleotides.

In a preferred embodiment the non extensible moiety is a peptide nucleic acid (PNA), preferably having at least 10 nucleotides.

DNA and RNA have a deoxyribose and ribose sugar backbone, respectively, whereas PNA's backbone is composed of repeating N-(2-aminoethyl)-glycine units linked by peptide bonds. Purine and pyrimidine bases are linked to the backbone by methylene carbonyl bonds. PNAs are depicted like peptides, with the N-terminus at the first (left) position and the C-terminus at the right. Since the backbone of PNA contains no charged phosphate groups, the binding between PNA/DNA strands is stronger than between DNA/DNA strands due to the lack of electrostatic repulsion.

In a most preferred embodiment said peptide nucleic acid (PNA) comprises a sequence of bases capable of hybridizing with the region including the putative point mutation resulting in double strand structures having respectively a melting temperature (Tm) = X with the wild type sequence and melting temperature (Tm) = Y with the mutant sequence, where Y< Incubation Temperature ≤ X and X is at least 5°C higher than Y. In a preferred embodiment the non extensible moiety is a self-annealed non extensible primer, comprising a central loop sequence able to selectively recognize and hybrizide to the region comprising the wild type sequence of the nucleic acid molecule, a 5' end sequence and a 3' end sequence, said 5' end and said 3' end sequences being complementary to each other to form a stem, so that said central loop sequence has a higher hybridization affinity to the region comprising the wild type sequence of the nucleic acid molecule than the hybridization affinity of the 5' end sequence to the 3' and sequence, so that it results in annealing and blocking of the wt sequence.

In a preferred embodiment the DNA polymerase with strand displacement activity is the Bst large fragment polymerase, or one of or a combination of: Bca (exo-), Vent, Vent (exo-), Deep Vent, Deep Vent (exo-), Φ29 phage, MS-2 phage, Z-Taq, KOD, Klenow fragment.

In a preferred embodiment the constant temperature is comprised between 62°C and 67°C.

In a preferred embodiment the signal indicative of amplification of the nucleotide molecule comprising the point mutation is detected by turbidimetry. Alternatively the signal indicative of amplification of the nucleotide molecule comprising the point mutation is detected by fluorescence.

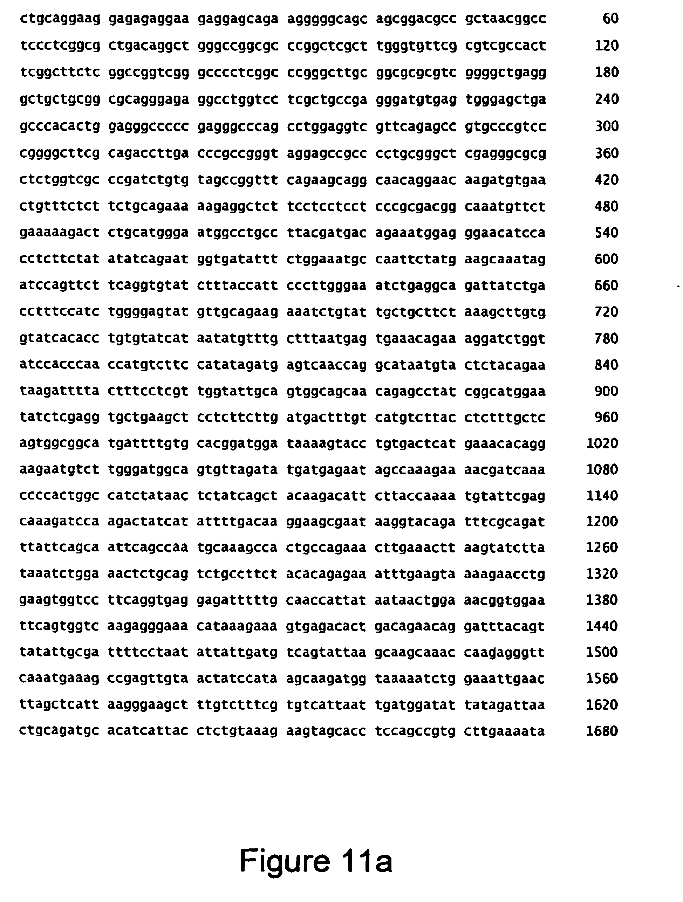

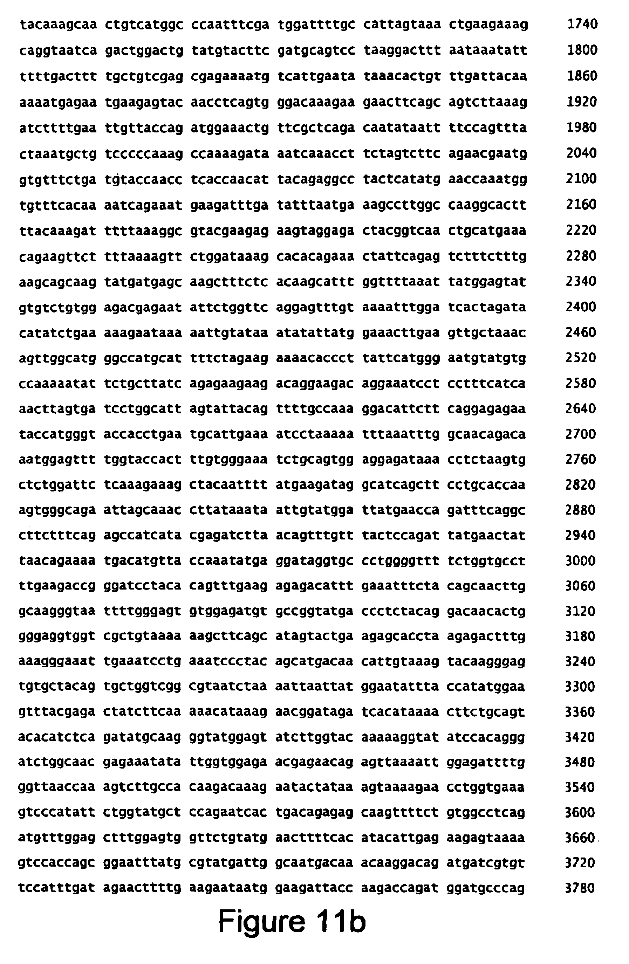

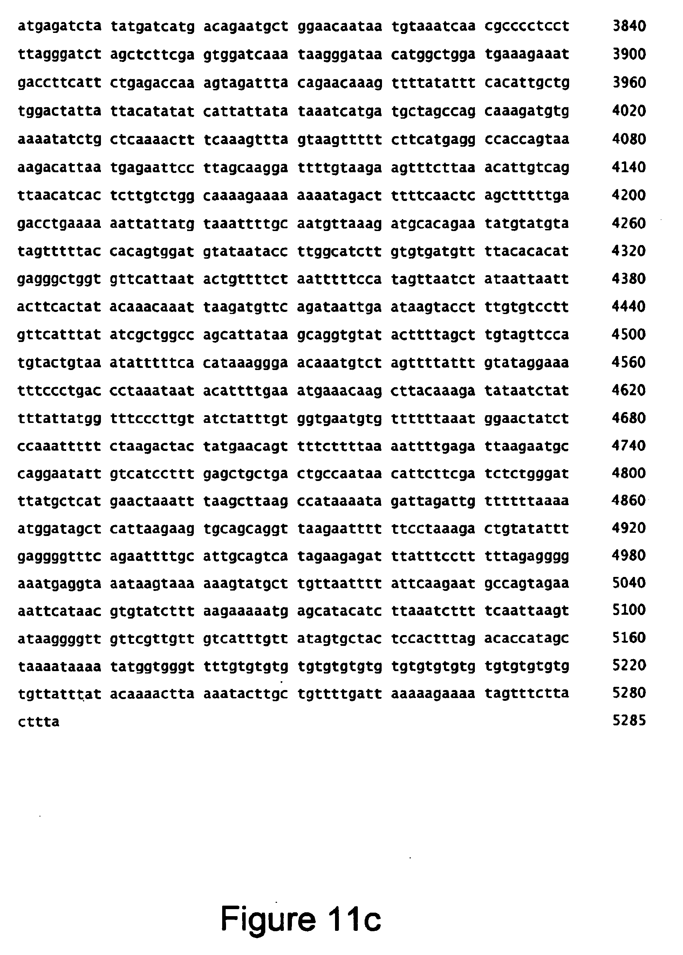

In a preferred embodiment the nucleic acid molecule comprises the region of the human JAK2 gene (GenBank accession no. NM_004972 illustrated in Figure 11a,b,c), putatively having the point mutation, guanine-to-thymidine substitution at base 1849 (G1489T). Preferably F2 and F1c are in position 1730-1750 and 1770-1795 respectively of the NM_004972 gene sequence; B2 is in position 1862-1884 of the NM_004972 gene sequence and B1c is in position 1810-1840 of the NM_004972 gene sequence. Most preferably the primers have the following sequences:
F3 5' - GCATCTTTATTATGGCAGAGAG- 3'; (Seq Id No.1)
B3 5'-TGCTCTGAGAAAGGCATTA- 3'; (Seq Id No. 2)
FIP 5'-GCTGCTTCAAAGAAAGACTAAGGAAATGGACAACAGTCAAACAAC -3'; (Seq Id No. 3)
BIP 5'-GCTTTCTCACAAGCATTTGGTTTTAAATTAGCCTGTAGTTTTACTTACTCTC-3' (Seq Id No. 4)

In a preferred embodiment the non extensible moiety is a PNA molecule, preferably having the structure: ^{NH2}GAGTATGTGTCTGTGGA^{CONH2}.

The method of the invention is applied also to other genes responsible for a pathology or an alteration, as i.e. kRAS, EGFR, and to SNPs.

The invention will be described with reference to specific not limiting examples, including the following figures:
**Figure 1****. LAMP principle (prior art)**
   The basic reaction is performed by 4 primers specific for 6 regions of a target genomic sequence . Internal primers anneal and extend on the target; the product is displaced in two steps by external primers (F3, B3) and is shaped as a double stem-loop structure *(starting structure)* (panel A). The starting structure is simultaneously amplified from its free 3' and by another internal primer (panel B). DNA concatamers built by inverted repeats of the initial module are progressively synthesized in an exponential fashion (panel C).

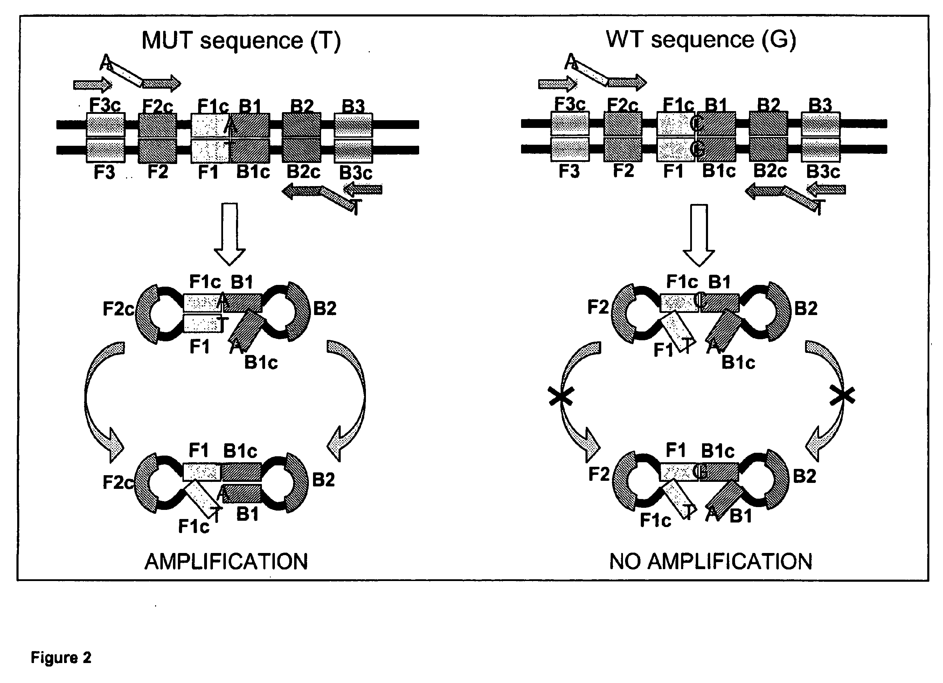

**Figure 2****. LAMP "DUMB-BELL strategy" principle (not working control)**
   The primers set is designed with the F1 c and B1 c region complementary respectively to one base upstream and one base downstream the nucleotide of interest in position 1849. Furthermore, the 5' end base of FIP and BIP is specific for the mutated nucleotide of JAK2 and both inner primers have a mismatched base at the third base from 3' end. When the dumb-bell structure is formed, If the target in the reaction is WT, the mutant specific F1c and B1c will not anneal at its 3' end resulting in no amplification. Differently, if mutant sequences are present in solution, the mutant specific F1c and B1c will perfectly anneal, becoming extensible by the polymerase.

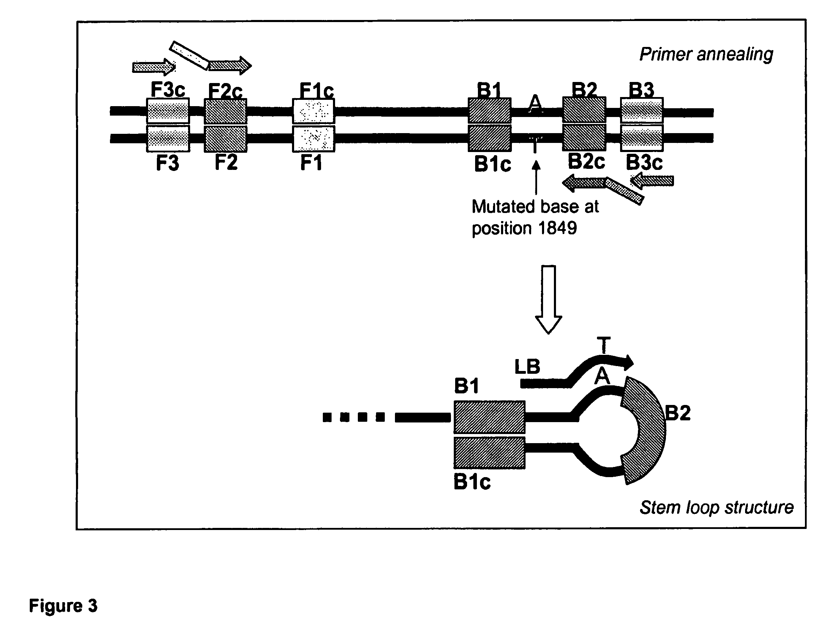

**Figure 3****. LAMP "Allele specific Loop primer extension strategy" principle**
   The only one loop primer in reaction has the last base in the 3' end complementary to the mutant nucleotide T at position 1849 of the JAK2 gene. It also presents a mismatched base in the third base from the 3' end. If the target in the reaction is WT, the mutant- specific loop primer will not anneal at its 3' end resulting in no amplification. Differently, if mutant sequences are present in solution, the mutant specific loop primer will perfectly anneal, becoming extensible by the polymerase.

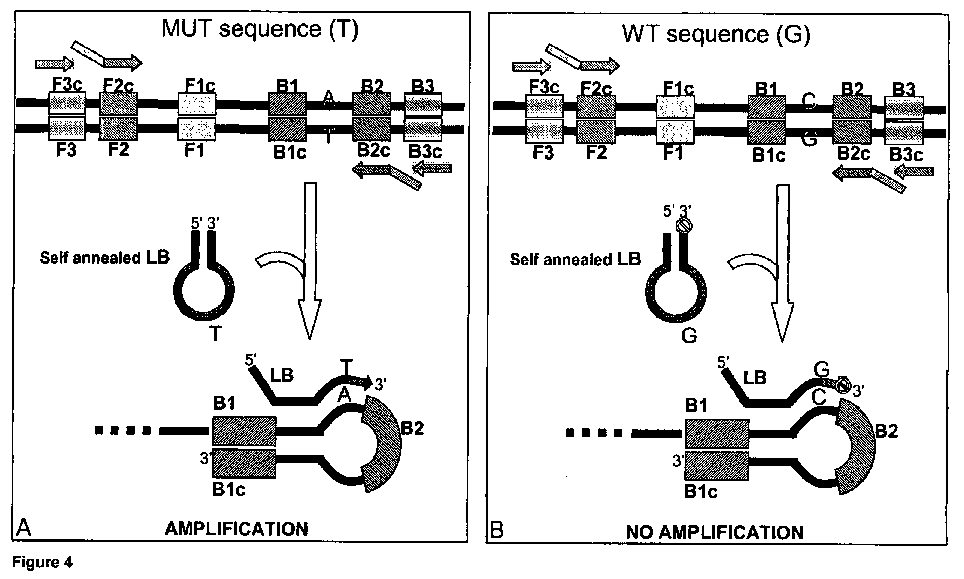

**Figure 4****. LAMP "Self-annealed Loop primers strategy" principle**
   Universal (mutant insensitive) set of primers comprising F3, B3, FIP and BIP to obtain a dumb-bell presenting the putative mutated nucleotide in the loop region. A particular loop primer designed to recognize the mutated base in the single strand dumb-bell structure is included, together with another modified loop primer complementary to the JAK2 wild type sequence and with the 3' end not extensible. When the mutated JAK2 sequence is present (panel A), the modified mutant loop primer breaks its internal structure to anneal to the target, being consequently extensible by the polymerase: the amplification can proceed. When the wt sequence is present in the sample (panel B), the modified wt loop primer anneals to the wt target resulting in no amplification of the wt sequences and avoiding the aspecific binding of mutant loop primer ("silencing" effect).

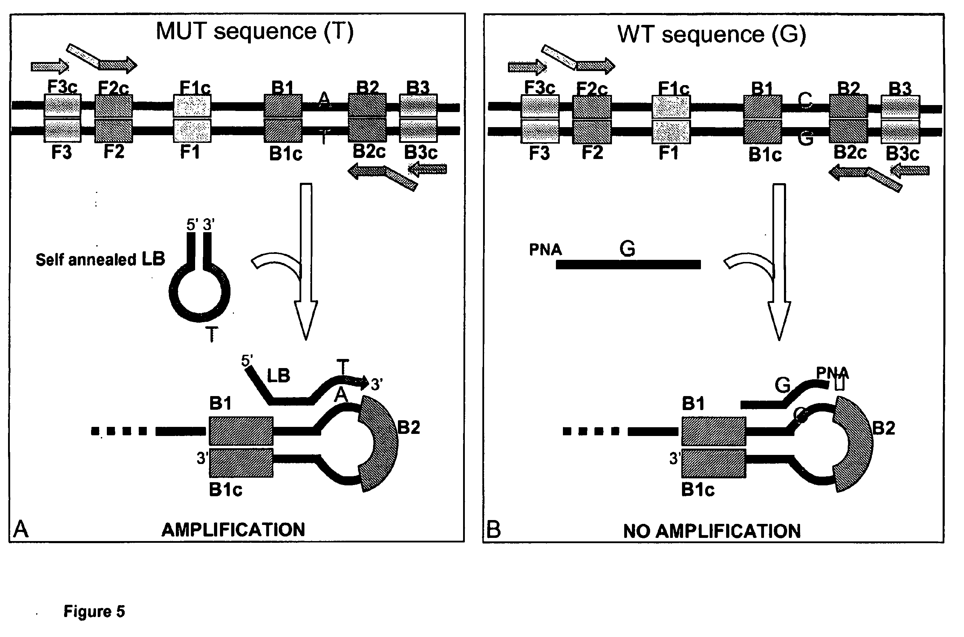

**Figure 5****. LAMP "Mutant Self-annealed Loop primer with PNA strategy" principle**
   Universal (mutant insensitive) set of primers comprising F3, B3, FIP and BIP to obtain a dumb-bell presenting the putative mutated nucleotide in the loop region. A particular self annealed-loop primer designed to recognize the mutated base in the single strand dumb-bell structure is included, together with a PNA probe. The PNA is designed to be complementary to the loop region comprised between B2 and B1 c presenting the WT nucleotide. It forms a stable duplex only with the wt complementary sequence, preventing the annealing and extension of the mut-self-annealed loop primer and therefore suppressing the amplification (panel B). It does not anneal to the mut JAK2 sequence thanks to the lower affinity (panel A).

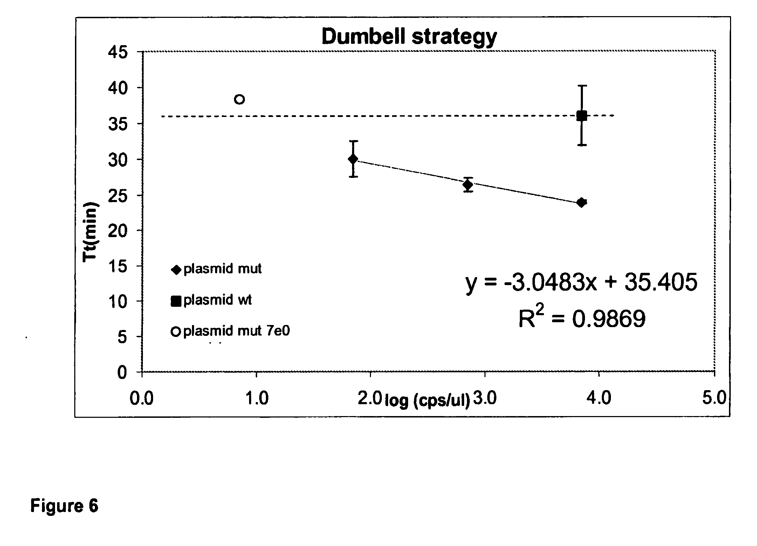

**Figure 6****. sensitivity of LAMP "DUMB-BELL strategy"**
   The reaction has been conducted on 7 e3 cps/ul wild type plasmid (square), on no-target control and on serial dilutions of mutant plasmid in water (from 7 e3 to 7 e1 cps/ul, rhomboidal points, and 7 e0 cps/ul, circle point). Each samples has been tested in triplicate. The error bars represent one standard deviation. The method amplify the specific target before the aspecific one until 7 e1 cps/ul concentration. The assay shows linearity between 7 e3 and 7 e1 cps/ul mutant sample.

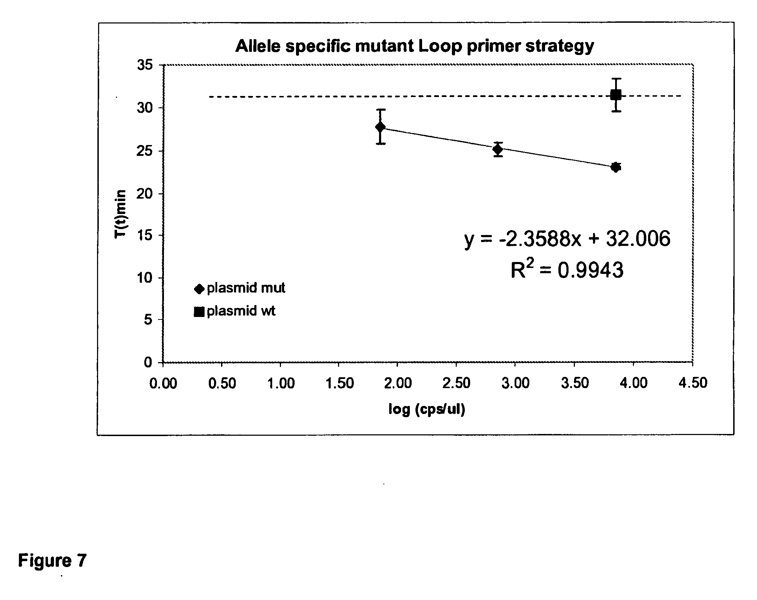

**Figure 7****. sensitivity of LAMP "Allele specific mutant loop primer"**
   The reaction has been conducted on 7 e3 cps/ul wild type plasmid (square point), on no-target control and on serial dilutions of mutant plasmid in water (from 7 e3 to 7 e1 cps/ul, rhomboidal points) . Each sample has been tested in triplicate. The error bars represent one standard deviation. The method amplify the specific target before the aspecific one until 7 e2 cps/ul concentration. The assay shows linearity between 7 e3 and 7 e1 cps/ul mutant sample.

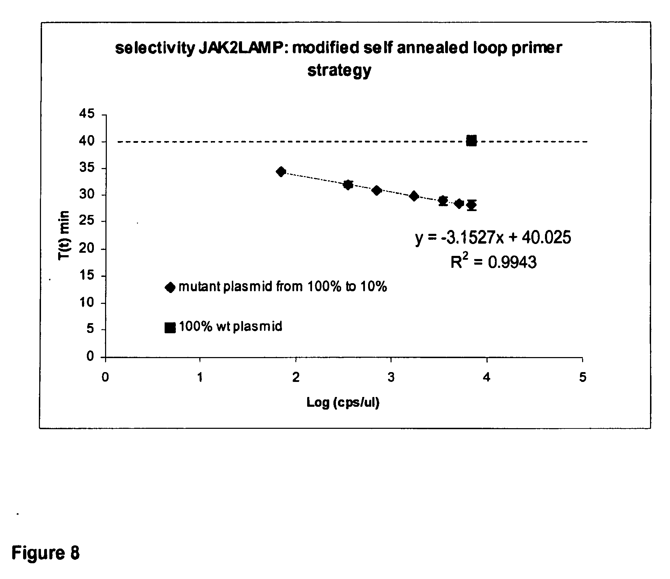

**Figure 8****. Selectivity of LAMP "self-annealed loop primer"**
   The reaction has been conducted on 7 e3 cps/ul wild type plasmid (square point), on no-target control and on serial dilutions of mutant plasmid in wild type plasmid, in proportions from 75% to 1%, 35000 cps total amount of DNA per reaction. Each sample has been tested in triplicate. The error bars represent one standard deviation. The method amplify the specific target before the aspecific one until 1 % dose (350 cps mut plasmid plus 34650 cps wt plasmid). The assay shows linearity between 100% and 1% mutant sample in wt background.

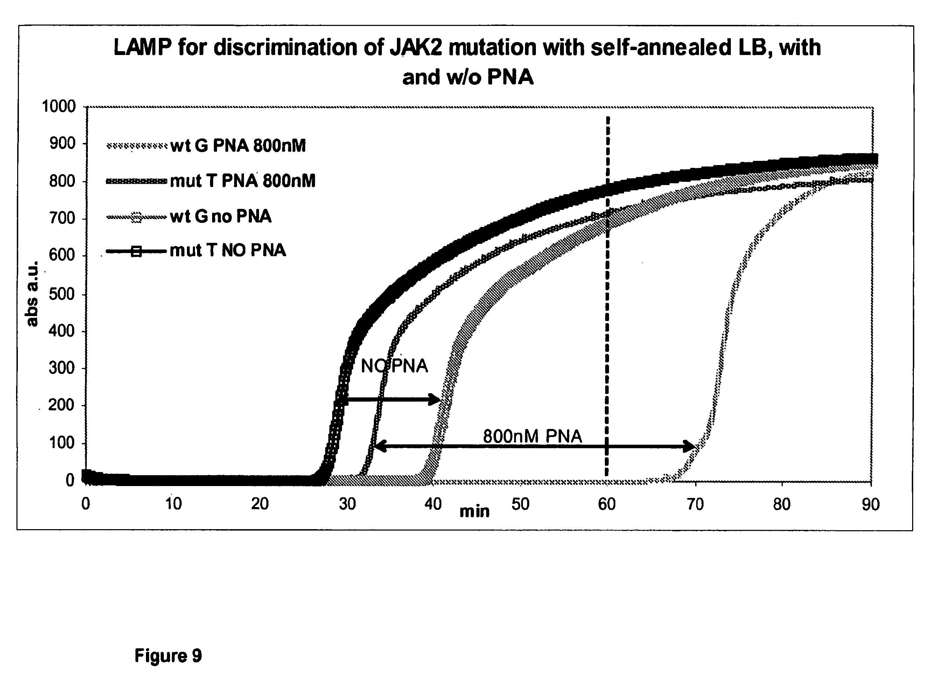

**Figure 9****. LAMP "self-annealed loop primer with PNA"**
   Test of the assay in presence and absence of PNA on mutated and wt plasmid (35000 cps each).In absence of PNA the WT plasmid is aspecifically amplified by the self-annealed mutant loop primer, with a delay of 5min in respect to the specific mutated target. In presence of PNA, the WT plasmid is not amplified within one hour reaction by the self- annealed mutant loop primer. The amplification of the mutant plasmid is delayed of about 5 minutes. The PNA forms a stable duplex only with the wt complementary sequence preventing the annealing and extension of the mut-self-annealed loop primer and therefore suppressing the amplification within 1 hour of reaction.

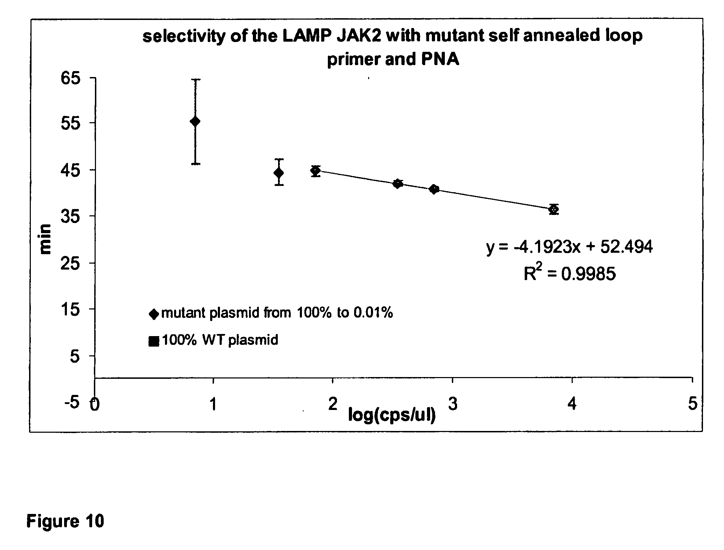

**Figure 10****. Selectivity of LAMP "self-annealed loop primer with PNA"**
   Test of the assay on mutated plasmid (350000 cps), wild type plasmid (350000 cps) and on mutated plasmid serially diluted into wt plasmid in 1, 0.5, 0.1, 0.05 and 0.01% proportion. Error bars correspond to 1 standard deviation. The WT sample (350000 cps wt plasmid) is not amplified in one hour reaction. The specific mutant target is detected down to 0.01% mutant sequences in wt (35 copies tot mutant plasmid in 349650 copies of wt plasmid). The assay is linear down to 0.1 % MUT (350 copies tot mutant plasmid in 349650 copies of wt plasmid).
Figure 11a,b,c illustrates the human JAK2 gene sequence available under GenBak accession No. NM_004972 and designated as SEQ ID No. 23 in the Sequence listing.

### EXAMPLE 1 - Materials, methods and results of the JAK2- modified- LAMP "dumb-bell strategy"

### Reagents

JAK2 plasmids were synthesized by the supplier GeneArt (Regensburg, Germany) to contain the wild type or the mutant JAK2 sequence. In details:
- JAK2 plasmid which Insert corresponds to the sequence 1689-1722 of MN_004972, including a G base at nucleotide1849, referred to as *"wt plasmid";*
- JAK2 plasmid which Insert corresponds to the sequence 1689-1722 of MN_004972 cloned, including a T base at nucleotide1849, referred to as *"mut plasmid".*

Primers: synthesized by the supplier Eurofins MWG Operon (Ebersberg, Germany) referred to as *"primers"*:
- GA211 (F3) 5'GTCAAACAACAATTCTTTGTACT 3' (Seq Id No. 5)
- GA212 (B3) 5'AGCTGTGATCCTGAAACTG 3' (Seq Id No. 6)
- GA216(FIP)
   5'AATATACTCCATAATTTAAAACCAAATGCTTTCTTTCTTTGAAGCAGCAAGT 3' (Seq Id No. 7)
- GA220(BIP)
   5'TTTTGTGGAGACGAGAGTAAGTAAAACTACATAAACAAAAACAGATGCTCTGA 3' (Seq Id No. 8)
- GA221 (LF) 5' GTGAGAAAGCTTGCTCATCAT 3' (Seq Id No. 9)
- GA222 (LB) 5' AGGCTTTCTAATGCCTTTC 3' (Seq Id No. 10)
Reaction buffer: 100mM Tris HCl pH 8.8, 50mM KCl, 40mM MgSO4, 50mM (NH4)2SO4, 0.5% Tween, 5% DMSO *"buffer 5x"*
dNTPs mix 25mM (Fermentas), *"dNTPs"*
Bst Large Fragment polymerase 8U/ul (New England Biolabs), *"Polymerase"*
Sterile apyrogen water (SALF Spa), *"ddw"*

### Procedure

### Sample preparation

Prepare reaction mix as follows: 0.2µM outer primers (F3 and B3), 1.6µM inner primers (FIP and BIP), 0.8uM loop primers (LF and LB), 1x buffer solution, 1.4mM dNTPs mix, 8 U Bst Polymerase. Final volume of the reaction mix must be 4/5 of the total reaction volume (i.e. 20µl reaction mix + 5µl sample). Always keep reagents on ice. Prepare the mix for at least 17 samples, comprising 3 negative control (7e3 cps/ul wild type plasmid), 12 positive control (3 samples 7e3 cps/ul mutant plasmid, 3 samples 7e2 cps/ul mutant plasmid , 3 samples 7e1 cps/ul mutant plasmid, 3 samples 7e0 cps/ul mutant plasmid) 1 no-target control.

**Table 1- sample mix composition**

| Sample tube | 1-3 | 4-6 | 7-9 | 10-12 | 13-15 | 16-19 |
|---|---|---|---|---|---|---|
| Target to be add (5µl) | Wt plasmid 7e3 cps/µl | Mut plasmid 7e3 cps/µl | Mut plasmid 7e2 cps/µl | Mut plasmid 7e1 cps/µl | Mut plasmid 7e0 cps/µl | |
| GA 211 100µM | 0.05 | 0.05 | 0.05 | 0.05 | 0.05 | 0.05 |
| GA212 100µM | 0.05 | 0.05 | 0.05 | 0.05 | 0.05 | 0.05 |
| GA 221 100µM | 0.2 | 0.2 | 0.2 | 0.2 | 0.2 | 0.2 |
| GA 221 100µM | 0.2 | 0.2 | 0.2 | 0.2 | 0.2 | 0.2 |
| GA 216 100µM | 0.4 | 0.4 | 0.4 | 0.4 | 0.4 | 0.4 |
| GA 220 100µM | 0.4 | 0.4 | 0.4 | 0.4 | 0.4 | 0.4 |
| Buffer LAMP 5x | 2.5 | 2.5 | 2.5 | 2.5 | 2.5 | 2.5 |
| Bst Polymerase 8U/µl | 1 | 1 | 1 | 1 | 1 | 1 |
| dNTPs 25mM | 1.4 | 1.4 | 1.4 | 1.4 | 1.4 | 1.4 |
| Ddw to 20 µl | 13.18 | 13.18 | 13.18 | 13.18 | 13.18 | 13.18 |

Dispense 20µl of reaction mix in the strip. Keep the strips on ice. Always keep the reaction mix on ice from now on.

Prepare serial dilutions of the target (*"target dilutions"*) from shipped solution (wt plasmid and mut plasmid). Shipped solution is a 7*10¹⁰ copies/µl. Dilute initially the mutant plasmid to a 7*10⁴ copies/µl in Tris 10mM, then dilute serially to 7e3 cps/µl, 7e2 cps/µl, 7e1 cps/µl and 7e0 cps/µl in Tris 10mM. Dilute the wt plasmid to 7*10³ copies/µl in Tris 10mM.

Add 5µl of target dilutions to the strips, in triplicate. Add 5µl of the target dilutions starting from the less concentrated one to the most concentrated one. Close all the tubes.

### Reaction

The reaction follows the method scheme of Figure 1 and 2.

Program the turbidimeter (Teramecs) for incubation at constant temperature and real time monitoring of turbidity, in order to obtain a constant reaction temperature of 66°C for 1 hour.

Put the strips in the instrument immediately before the beginning of the programs. Start the program.

### Data analysis

Analyze the variation of absorbance in terms of a.u. (arbitrary units of absorbance) to find the threshold time for each sample analyzed. The threshold time is the minute at which the sample absorbance, after baseline subtraction, reaches the arbitrary unit value representing the threshold (in this case 0.1 a.u.). The threshold time reached by each samples is correlated with its Log of DNA copies/µl.

### Results

"LAMP JAK2 Dumb-bell strategy" is based on the Eiken LAMP method for SNP detection (described in EP 1231281, 20, 21, 22, as well as on http://loopamp.eiken.co.jp/e/lamp/snps_anim.html). As shown in Figure 2, the primers set is designed with the F1c and B1c region complementary respectively to one base upstream and one base downstream the nucleotide of interest in position 1849. Furthermore, the 5' end base of FIP and BIP is specific for the mutated nucleotide of JAK2 and both inner primers have a mismatched base at the third base from 3' end. When the dumb-bell structure is formed, if the target in the reaction is WT, the mutant specific F1c and B1c will not anneal at its 3' end resulting in no amplification because the mismatch at its 3'end should not be extensible. Differently, if mutant sequences are present in solution, the mutant specific F1c and B1c will perfectly anneal, becoming extensible by the polymerase.

A shown in Figure 6, the assay was able to detect the mutant plasmid from 7e3 cps/µl to 7e0 cps/µl (35 copies tot mutant plasmid). It amplifies aspecifically the wt plasmid 7e3 cps/µl, not distinguishing the lower concentrations of mut plasmid from the aspecific target. The level of selectivity should be less than 1%, which is the limit shown by the other techniques in literature. With this approach we don't have any clear advantage.

### EXAMPLE 2 - Materials, methods and results of the JAK2- modified- LAMP "Allele specific Loop primer extension strategy"

### Reagents

JAK2 plasmids were synthesized by the supplier GeneArt (Regensburg, Germany) to contain the wild type or the mutant JAK2 sequence. In details:
- which Insert corresponds to the sequence 1689-1722 of MN_004972, including a G base at nucleotide1849, referred to as *"wt plasmid"*
- which Insert corresponds to the sequence 1689-1722 of MN_004972 cloned, including a T base at nucleotide1849, referred to as *"mut plasmid"*

Primers: synthesized by Eurofins MWG Operon, referred to as *"primers"*:
- JAKR5 (F3) 5' TCTATAGTCATGCTGAAAGTAGGAG 3' (Seq Id No. 11)
- JAKR2 (B3) 5' AAGGCATTAGAAAGCCTGTAGT 3' (Seq Id No. 12)
- JAKR7 (FIP)
   5'ACAAAGAATTGTTGTTTGACTGTTGTCCATTGCATCTTTATTATGGCAGAGAGAA3' (Seq Id No. 13)
- JAKR8 (BIP)
   5' AGTCTTTCTTTGAAGCAGCAAGTATGATGTTACTTACTCTCGTCTCCACAGA 3' (Seq Id No. 14)
- JAKR9 (LB) 5' AGCATTTGGTTTTAAATTATGGAGTAGGTT 3' (Seq Id No. 15) The underlined base corresponds to a mismatched nucleotide. The bold base corresponds to the mutated nucleotide at position 1849 of the JAK2 gene.
Reaction buffer: 100mM Tris HCl pH 8.8, 50mM KCI, 40mM MgSO4, 50mM (NH4)2SO4, 0.5% Tween, *"buffer 5x"*
dNTPs mix 25mM (Fermentas), "*dNTPs*"
Bst Large Fragment polymerase 8U/ul (New England Biolabs), *"Polymerase"*
Sterile apyrogen water (SALF Spa), *"ddw"*

### Procedure

### Sample preparation

Stock the primers in aliquots. It is better to store stock solutions at -20°C, while working dilutions should be stored at 4°C.

Prepare reaction mix as follows: 0.2µM outer primers (F3 and b3), 1.6µM inner primers (FIP and BIP), 0.8uM loop primer (LB), 1x buffer solution, 1.4mM dNTPs mix, 8 U Bst Polymerase. Final volume of the reaction mix must be 4/5 of the total reaction volume (i.e. 20µl reaction mix + 5µl sample). Always keep reagents on ice. Prepare the mix for at least 14 samples, comprising 3 negative controls (7e3 cps/ul wild type plasmid), 9 positive control (3 samples 7e3 cps/ul mutant plasmid, 3 samples 7e2 cps/ul mutant plasmid , 3 samples 7e1 cps/ul mutant plasmid) 1 no-target control.

**Table 2- sample mix composition**

| Sample tube | 1-3 | 4-6 | 7-9 | 10-12 | 13-15 | 16-19 |
|---|---|---|---|---|---|---|
| Target to be add (5µl) | Wt plasmid 7e3 cps/µl | Mut plasmid 7e3 cps/µl | Mut plasmid 7e2 cps/µl | Mut plasmid 7e1 cps/µl | Mut plasmid 7e0 cps/µl | |
| JAKR5 100µM | 0.05 | 0.05 | 0.05 | 0.05 | 0.05 | 0.05 |
| JAKR2 100µM | 0.05 | 0.05 | 0.05 | 0.05 | 0.05 | 0.05 |
| JAKR7 100µM | 0.4 | 0.4 | 0.4 | 0.4 | 0.4 | 0.4 |
| JAKR8 100µM | 0.4 | 0.4 | 0.4 | 0.4 | 0.4 | 0.4 |
| JAKR9 100µM | 0.2 | 0.2 | 0.2 | 0.2 | 0.2 | 0.2 |
| Buffer LAMP 10x | 2.5 | 2.5 | 2.5 | 2.5 | 2.5 | 2.5 |
| Bst Polymerase 8U/µl | 1 | 1 | 1 | 1 | 1 | 1 |
| dNTPs 25mM | 1.4 | 1.4 | 1.4 | 1.4 | 1.4 | 1.4 |
| Ddw to 20 µl | 14 | 14 | 14 | 14 | 14 | 14 |
| | | | | | | |

Dispense 20µl of reaction mix in the strip. Keep the strips on ice. Always keep the reaction mix on ice from now on.

Prepare serial dilutions of the target (*"target dilutions"*) from shipped solution (wt plasmid and mut plasmid). Shipped solution is a 7*10¹⁰ copies/µl. Dilute initially the mutant plasmid to a 7*10⁴ copies/µl in Tris 10mM, then dilute serially to 7e3 cps/µl, 7e2 cps/µl, 7e1 cps/µl in Tris 10mM. Dilute the wt plasmid to 7*10³ copies/µl in Tris 10mM. Add 5µl of target dilutions to the strips, in triplicate. Add 5µl of the target dilutions starting from the less concentrated one to the most concentrated one. Close all the tubes.

### Reaction

The reaction follows the method scheme of Figure 3.

Program the turbidimeter (Teramecs) for incubation at constant temperature and real time monitoring of turbidity
in order to obtain a constant reaction temperature of 65°C for 1 hour.

Put the strips in the instrument immediately before the beginning of the programs. Start the program.

### Data analysis

Analyze the variation of absorbance in terms of a.u. to find the threshold time for each sample analyzed. The threshold time is the minute at which the sample absorbance, after baseline subtraction, reaches the arbitrary unit value representing the threshold (in this case 0.1 a.u.). The threshold time reached by each samples is correlated with its Log of DNA copies/µl.

### Results

This approach consists of a selective mutant amplification based on a mutant- specific loop primer (Figure 3). We designed a universal (mutant insensitive) set of primers comprising F3, B3, FIP and BIP to obtain a dumb-bell presenting the putative mutated nucleotide in the loop region comprised between B2 and B1c. We therefore designed only one loop primer presenting the last base in the 3' end complementary to the mutant nucleotide T at position 1849 of the JAK2 gene. It also presents a mismatched base in the third base from the 3' end.

If the target in the reaction is WT, the mutant- specific loop primer will not anneal at its 3' end resulting in no amplification. Differently, if mutant sequences are present in solution, the mutant specific loop primer will perfectly anneal, becoming extensible by the polymerase.

We tested this assay on the mutant plasmid from 7e3 cps/µl to 7e1 cps/µl (35000 and 350 copies tot mutant plasmid) and on the aspecific wt plasmid (7 e3 cps/µl), all in triplicate. The assay amplifies aspecifically the wt plasmid 7e3 cps/µl, not distinguishing the 7e1 cps/µl concentrations of mut plasmid from the aspecific target. The level of selectivity should be less than 1%, which is the limit shown by the other techniques in literature. With this approach we don't have any clear advantage. (Figure 7).

### EXAMPLE 3 - Materials, methods and results of the JAK2- modified- LAMP "Self-annealing Loop primer strategy"

### Reagents

JAK2 plasmids were synthesized by the supplier GeneArt (Regensburg, Germany) to contain the wild type or the mutant JAK2 sequence. In details:
- which Insert corresponds to the sequence 1689-1722 of MN_004972, including a G base at nucleotide1849, referred to as *"wt plasmid"*
- which Insert corresponds to the sequence 1689-1722 of MN_004972 cloned, including a T base at nucleotide1849, referred to as *"mut plasmid"*

Primers: synthesized by Eurofins MWG Operon, referred to as *"primers"*.
- GA231 (F3) 5' GCATCTTTATTATGGCAGAGAG 3' (Seq Id No. 16)
- GA232 (B3) 5' TGCTCTGAGAAAGGCATTA 3' (Seq Id No. 17)
- GA233 (FIP)
   5' GCTGCTTCAAAGAAAGACTAAGGAAATGGACAACAGTCAAACAAC 3' (Seq Id No. 18)
- GA234 (BIP)
   5' GCTTTCTCACAAGCATTTGGTTTTAAATTAGCCTGTAGTTTTACTTACTCTC 3' (Seq Id No. 19)
- GA235 (LF) 5'GTCTCCACTGGAGTATGTGTCTGTGGAGAddC3' (Seq Id No. 20) the underlined base is the wild type nucleotide in position 1849 of the JAK2 gene. ddC stands for not-extensible dideoxy-cytosine.
- GA236 (LB) 5'GTCTCCACTGGAGTATGTTTCTGTGGAGAC 3' (Seq Id No. 21) the underlined base is the mutant nucleotide in position 1849 of the JAK2 gene.
Reaction buffer: 100mM Tris HCl pH 8.8, 50mM KCl, 40mM MgSO₄ 50mM (NH₄)₂SO₄ 0.5% Tween, *"buffer 5x*" dNTPs mix 25mM (Fermentas), "*dNTPs*"
Bst Large Fragment polymerase 8U/ul (New England Biolabs), *"Polymerase"*
Sterile apyrogen water (SALF Spa), *"ddw"*

### Procedure

### Sample preparation

Stock the primers in aliquots. It is better to store stock solutions at -20°C, while working dilutions should be stored at 4°C.

Prepare reaction mix as follows: 0.2µM outer primers (F3 and B3), 1.6µM inner primers (FIP and BIP), 0.8uM both self-annealed loop primers (not-extensible LF and LB), 1x buffer solution, 1.4mM dNTPs mix, 8 U Bst Polymerase. Final volume of the reaction mix must be 4/5 of the total reaction volume (i.e. 20µl reaction mix + 5µl sample). Always keep reagents on ice. Prepare the mix for at least 26 samples, comprising 3 negative controls (100% wild type plasmid, 7e3 cps/ul), 21 positive control (3 samples 100% (7e3 cps/ul) mutant plasmid, 3 samples 75% mutant plasmid diluted in wt plasmid, 3 samples 50% mutant plasmid diluted in wt plasmid, 3 samples 25% mutant plasmid diluted in wt plasmid, 3 samples 10% mutant plasmid diluted in wt plasmid, 3 samples 10% mutant plasmid diluted in wt plasmid, 3 samples 5% mutant plasmid diluted in wt plasmid, 3 samples 1% mutant plasmid diluted in wt plasmid, and one no target control.

**Table 3- sample mix composition**

| Sample tube | 1-3 | 4-6 | 7-9 | 10-12 | 13-15 | 16-19 |
|---|---|---|---|---|---|---|
| Target to be add (5µl) | Wt plasmid 7e3 cps/µl | Mut plasmid 7e3 cps/µl | Mut plasmid 7e2 cps/µl | Mut plasmid 7e1 cps/µl | Mut plasmid 7e0 cps/µl | |
| GA231 100µM | 0.05 | 0.05 | 0.05 | 0.05 | 0.05 | 0.05 |
| GA232 100µM | 0.05 | 0.05 | 0.05 | 0.05 | 0.05 | 0.05 |
| GA233 100µM | 0.4 | 0.4 | 0.4 | 0.4 | 0.4 | 0.4 |
| GA234 100µM | 0.4 | 0.4 | 0.4 | 0.4 | 0.4 | 0.4 |
| GA235 100µM | 0.2 | 0.2 | 0.2 | 0.2 | 0.2 | 0.2 |
| GA236 100µM | 0.2 | 0.2 | 0.2 | 0.2 | 0.2 | 0.2 |
| Buffer LAMP 10x | 2.5 | 2.5 | 2.5 | 2.5 | 2.5 | 2.5 |
| Bst Polymerase 8U/µl | 1 | 1 | 1 | 1 | 1 | 1 |
| dNTPs 25mM | 1.4 | 1.4 | 1.4 | 1.4 | 1.4 | 1.4 |
| Ddw to 20 µl | 13.8 | 13.8 | 13.8 | 13.8 | 13.8 | 13.8 |

Dispense 20µl of reaction mix in the strip. Keep the strips on ice. Always keep the reaction mix on ice from now on.

Prepare serial dilutions of the target (*"target dilutions"*) from shipped solution (wt plasmid and mut plasmid). Shipped solution is a 7*10¹⁰ copies/µl. Dilute initially the mutant plasmid to a 7*10⁴ copies/µl in Tris 10mM, then dilute serially the mutant plasmid in wt plasmid to obtain the following concentrations of mutant sequences in wild type background: 75%, 50%, 25%, 10%, 5%, 1% (total amount per tube, 7e3 cps/µl). Add 5µl of target dilutions to the strips, in triplicate. Add 5ul of the target dilutions starting from the less concentrated one to the most concentrated one. Close all the tubes.

### Reaction

The reaction follows the method scheme of Figure 4.

Program the turbidimeter (Teramecs) for incubation at constant temperature and real time monitoring of turbidity, in order to obtain a constant reaction temperature of 65°C for 1 hour.

Put the strips in the instrument immediately before the beginning of the programs. Start the program.

### Data analysis

Analyze the variation of absorbance in terms of a.u. to find the threshold time for each sample analyzed. The threshold time is the minute at which the sample absorbance, after baseline subtraction, reaches the arbitrary unit value representing the threshold (in this case 0.1 a.u.). The threshold time reached by each samples is correlated with its Log of DNA copies/µl.

### Results

This approach consists of a selective mutant amplification based on a particular loop primer design resulting in selective hybridization of such loop primer to the dumb-bell formed from the mutant sequence (Figure 4). We designed a universal (mutant insensitive) set of primers comprising F3, B3, FIP and BIP to obtain a dumb-bell presenting the putative mutated nucleotide in the loop region comprised between B1 and B2. Other experiments were performed presenting the putative mutated nucleotide in the loop region into B2 or comprised between B2 and B1c, with no relevant differences. We included in the primer set a particular loop primer presenting a 8-bases sequence region at its 5' end complementary to its own sequence in 3' end. Consequently, this special loop primer forms an intra-molecular hairpin structure in equilibrium with its open form at the reaction temperature (65°C).

When the mutated JAK2 sequence is present, this modified loop primer breaks its internal structure to anneal to the target, thanks to the thermodynamic equilibrium (Tm between primer and specific target = 65°C). The loop primer annealed to the specific mutated target is consequently extensible by the polymerase: the amplification can proceed.

When the wt sequence is present in the sample, the same loop primer (specific for the MUT JAK2 gene) presents a Tm with aspecific target ( 59°C) lower than the intra-molecular hairpin structure (65°C). This results in auto-sequestration of the modified loop primer that prefers to fold in the hairpin structure rather than to form a duplex with aspecific target, since the intramolecular forces are higher than the intermolecular ones. To limit the competition of the loop primer previously described for the wild type sequences likely to be present in large excess in the clinical sample, we added another modified loop primer characterized by a structure similar to the one previously described, but whit a sequence complementary to the JAK2 wild type sequence (with G base at position 1489).

The 3' end of this "competitor" loop primer is made not extensible by a modification (3' dideoxy). The task of this competitor is to "silence" the wt and allow the specific mutant primer to find its target.

When the "competitor" recognizes the specific wild type sequence, it breaks its intramolecular structure to anneal to the WT target, thanks to a higher affinity (Tm duplex wt target-wt modified loop primer= 67°C); The loop primer annealed to the wt target is not extensible, resulting in no amplification of the wt sequences. Since the reaction is conducted at constant temperature, the wt-loop primer will remain annealed to the wt sequences preventing aspecific annealing of the MUT loop primer.

Differently, the "competitor" presents a Tm with its aspecific (mutant) target ( 62°C) lower than the intra-molecular hairpin structure that it forms with itself (65°C). This results in auto-sequestration of the modified loop primer that prefers to fold in the hairpin structure rather than to form a duplex with the aspecific target, since the intramolecular forces are higher than the intermolecular ones.

The selectivity of this assay has been evaluated, performing the reaction on serial dilutions of mutant plasmid in wild type background (Figure 8). The selectivity achieved is significantly less than 1% (350 copies tot mutant plasmid in 34650 copies of wt plasmid). This approach has higher selectivity than the assays described in literature. The assay is linear between 100% mutant (35000 cps) and 1% mutant in 99% wild type (350 copies tot mutant plasmid in 34650 copies of wt plasmid). It allows detection and quantification of low percentage of mutant sequences in large amount of wt. It represents an improvement in respect of the other approaches shown in the previous slides and in respect to the methods described in literature.

### EXAMPLE 4 - Materials, methods and results of the JAK2- modified- LAMP "Self-annealing Loop primer strategy with PNA"

### Reagents

JAK2 plasmids were synthesized by the supplier GeneArt (Regensburg, Germany) to contain the wild type or the mutant JAK2 sequence. In details:
- which Insert corresponds to the sequence 1689-1722 of MN_004972, including a G base at nucleotide1849, referred to as *"wt plasmid"*
- which Insert corresponds to the sequence 1689-1722 of MN_004972 cloned, including a T base at nucleotide1849, referred to as *"mut plasmid"* Primers: synthesized by Eurofins MWG Operon, referred to as *"primers"*
- GA231 (F3) 5' GCATCTTTATTATGGCAGAGAG 3' (Seq Id No. 16)
- GA232 (B3) 5' TGCTCTGAGAAAGGCATTA 3' (Seq Id No. 17)
- GA233 (FIP)
- 5' GCTGCTTCAAAGAAAGACTAAGGAAATGGACAACAGTCAAACAAC 3' (Seq Id No. 18)
- GA234 (BIP)
   5'GCTTTCTCACAAGCATTTGGTTTTAAATTAGCCTGTAGTTTTACTTACTCTC 3' (Seq Id No. 19)
- GA236 (LB) 5' GTCTCCACTGGAGTATGTTTCTGTGGAGAC 3' (Seq Id No. 22)

The underlined base corresponds to the mutated nucleotide at position 1849 of the JAK2 gene.
PNA: Eurogentec, referred to as *"PNA" GM43* ^{NH2}GAGTATGTGTCTGTGGA^{CONH2}

The underlined base corresponds to the wild type nucleotide at position 1849 of the JAK2 gene.
Reaction buffer: 100mM Tris HCl pH 8.8, 50mM KCI, 40mM MgSO4, 50mM (NH4)2SO4, 0.5% Tween, *"buffer 5x"*
dNTPs mix 25mM (Fermentas), *"dNTPs"*
Bst Large Fragment polymerase 8U/µl (New England Biolabs), *"Polymerase"*
Sterile apyrogen water (SALF Spa), *"ddw"*

### Procedure

### Sample preparation

Stock the primers in aliquots. It is better to store stock solutions at -20°C, while working dilutions should be stored at 4°C

Prepare reaction mix as follows: 0.2µM outer primers (F3 and B3), 1.6µM inner primers (FIP and BIP), 0.8uM self-annealed loop primer specific for mutant JAK2 (LB), 0.8uM PNA, 1x buffer solution, 1.4mM dNTPs mix, 8 U Bst Polymerase. Final volume of the reaction mix must be 4/5 of the total reaction volume (i.e. 20µl reaction mix + 5µl sample). Always keep reagents on ice. Prepare the mix for at least 23 samples, comprising 3 negative controls (100% wild type plasmid, 7e4 cps/µl), 18 positive control (3 samples 100% mutant plasmid, 3 samples 1% mutant plasmid diluted in wt plasmid, 3 samples 0.5% mutant plasmid diluted in wt plasmid, 3 samples 0.1% mutant plasmid diluted in wt plasmid, 3 samples 0.05% mutant plasmid diluted in wt plasmid, 3 samples 0.01% mutant plasmid diluted in wt plasmid (total amount of DNA 7e4 cps/µl), and one no target control.

**Table 4- sample mix composition**

| Sample tube | 1-3 | 4-6 | 7-9 | 10-12 | 13-15 | 16-19 |
|---|---|---|---|---|---|---|
| Target to be add (5µl) | Wt plasmid 7e4 cps/µl | Mut plasmid 7e4 cps/µl | Mut plasmid 7e3 cps/µl | Mut plasmid 7e2 cps/µl | Mut plasmid 7e1 cps/µl | Mut plasmid 7e0 cps/µl |
| GA231 100µM | 0.05 | 0.05 | 0.05 | 0.05 | 0.05 | 0.05 |
| GA232 100µM | 0.05 | 0.05 | 0.05 | 0.05 | 0.05 | 0.05 |
| GA233 100µM | 0.4 | 0.4 | 0.4 | 0.4 | 0.4 | 0.4 |
| GA234 100µM | 0.4 | 0.4 | 0.4 | 0.4 | 0.4 | 0.4 |
| PNA 100µM | 0.2 | 0.2 | 0.2 | 0.2 | 0.2 | 0.2 |
| GA236 100µM | 0.2 | 0.2 | 0.2 | 0.2 | 0.2 | 0.2 |
| Buffer LAMP 10x | 2.5 | 2.5 | 2.5 | 2.5 | 2.5 | 2.5 |
| Bst Polymerase 8U/µl | 1 | 1 | 1 | 1 | 1 | 1 |
| dNTPs 25mM | 1.4 | 1.4 | 1.4 | 1.4 | 1.4 | 1.4 |
| Ddw to 20 µl | 13.8 | 13.8 | 13.8 | 13.8 | 13.8 | 13.8 |

Dispense 20µl of reaction mix in the strip. Keep the strips on ice. Always keep the reaction mix on ice from now on.

Prepare serial dilutions of the target *("target dilutions")* from shipped solution (wt plasmid and mut plasmid). Shipped solution is a 7*10¹⁰ copies/µl. Dilute initially the mutant plasmid to a 7*10⁴ copies/µl in Tris 10mM, then dilute serially the mutant plasmid in wt plasmid to obtain the following concentrations of mutant sequences in wild type background: 1%, 0.5%, 0.1%, 0.05%, 0.01% (total amount per tube, 7e4 cps/ul). Add 5µl of target dilutions to the strips, in triplicate. Add 5ul of the target dilutions starting from the less concentrated one to the most concentrated one. Close all the tubes.

### Reaction

The reaction follows the method scheme of Figure 5.

Program the turbidimeter (Teramecs) for incubation at constant temperature and real time monitoring of turbidity in order to obtain a constant reaction temperature of 65°C for 1 hour.

Put the strips in the instrument immediately before the beginning of the programs. Start the program.

### Data analysis

Analyze the variation of absorbance in terms of a.u. to find the threshold time for each sample analyzed. The threshold time is the minute at which the sample absorbance, after baseline subtraction, reaches the arbitrary unit value representing the threshold (in this case 0.1 a.u.). The threshold time reached by each samples is correlated with its Log of DNA copies/µl.

### Results

This approach consists of a selective mutant amplification based on a particular loop primer design resulting in selective hybridization of such loop primer to the dumb-bell formed from the mutant sequence (Figure 5). We designed a universal (mutant insensitive) set of primers comprising F3, B3, FIP and BIP to obtain a dumb-bell presenting the putative mutated nucleotide in the loop region comprised between B1 and B2. Other experiments were performed presenting the putative mutated nucleotide in the loop region into B2 or comprised between B2 and B1 c, with no relevant differences. We included in the primer set a particular loop primer presenting a 8-bases sequence region at its 5' end complementary to its own sequence in 3' end. Consequently, this special loop primer forms an intra-molecular hairpin structure in equilibrium with its open form at the reaction temperature (65°C).

When the mutated JAK2 sequence is present, this modified loop primer breaks its internal structure to anneal to the target, thanks to the thermodynamic equilibrium (Tm between primer and specific target = 65°C). The loop primer annealed to the specific mutated target is consequently extensible by the polymerase: the amplification can proceed.

When the wt sequence is present in the sample, the same loop primer (specific for the MUT JAK2 gene) presents a Tm with aspecific target ( 59°C) lower than the intra-molecular hairpin structure (65°C). This results in auto-sequestration of the modified loop primer that prefers to fold in the hairpin structure rather than to form a duplex with aspecific target, since the intramolecular forces are higher than the intermolecular ones. To further increase the discrimination capability of the LAMP system based on selective self-annealed loop primer, we added to the reaction mix a Peptide Nucleic Acid (PNA). PNAs are non-extensible and not-displaceable oligonucleotides where the ribose-phosphate backbone is replaced by (2- aminoethyl)-glycine units linked by amide bonds. Each base pairing DNA/PNA contributes to the stability of the duplex structure more than a regular base pairing DNA/DNA. Therefore a single mismatch in a PNA/DNA duplex results in a significant difference in Tm. A PNA probe fully complementary to the WT sequence of the JAK2 gene prevents annealing and extension of the mutant self-annealed primer, suppressing amplification. In presence of a single mismatch, PNA does not inhibit loop primer hybridization, which leads to amplification. Therefore PNA can be used to selectively block the Wt sequence present in the sample.

The PNA is designed to be complementary to the loop region comprised between B2 and B1c presenting the WT nucleotide. It forms a stable duplex only with the wt complementary sequence (Tm 65.7°C), preventing the annealing and extension of the mut-self-annealed loop primer and therefore suppressing the amplification. It does not anneal to the mut JAK2 sequence thanks to the lower affinity (Tm= 56°C).

The PNA principle has been tested performing the reaction on 7 e3 cps/ul wild type plasmid and on 7 e3 cps/ul mutant plasmid, in parallel in absence and presence of the PNA "wt blocker" probe (Figure 9). In absence of PNA the WT plasmid is aspecifically amplified by the self-annealed mutant loop primer, with a delay of 5min in respect to the specific mutated target. In presence of PNA, the WT plasmid is not amplified within one hour reaction by the self- annealed mutant loop primer. The amplification of the mutant plasmid is delayed of about 5 minutes. The PNA forms a stable duplex only with the wt complementary sequence preventing the annealing and extension of the mut-self-annealed loop primer and therefore suppressing the amplification within 1 hour of reaction.

The selectivity of this assay has been evaluated, performing the reaction on serial dilutions of mutant plasmid in wild type background (Figure 10). The selectivity achieved is less than 0.01 % (35 copies tot mutant plasmid in 34965 copies of wt plasmid). This approach has higher selectivity than the assays described in prior art, about 3 Logs more in respect to direct sequencing, RFLP and pyrosequencing and about 2 Logs in respect to ARMS, Real-time techniques and DNA-melting curve analysis. As to LAMP dumb-bell method , it is intrinsically not useful to detect single point mutations in a high background of wild type sequences.

The WT sample (35000 cps wt plasmid) is not amplified in one hour reaction. The specific mutant target is detected down to 0.01% mutant sequences in wt (35 copies tot mutant plasmid in 34965 copies of wt plasmid). This approach has a higher selectivity than the assays described in literature (about 2 Logs). The assay is linear down to 0.1 % MUT (350 copies tot mutant plasmid in 34650 copies of wt plasmid). It allows detection and quantification of low percentage of mutant sequences in large amount of wt. It represents a further improvement in respect of the other strategies described in this report and in respect to the methods described in literature.

### EXAMPLE 5 - LAMP "self-annealed loop primer with PNA"on clinical samples: comparison with ARMs

29 samples of DNA extracted from patients at Ospedali Riuniti di Bergamo were analyzed using JAK2 LAMP "self-annealed loop primer with PNA" strategy, as described in the EXAMPLE 4. The results obtained have been compared with the ones obtained at the hospital using the ARMs technology. The ARMS exploits the fact that oligonucleotide primers must be perfectly annealed at their 3' ends for a DNA polymerase to extend these primers during PCR. By designing oligonucleotide primers that match only the specific JAK2 point mutation ARMS can distinguish between wild type and mutant alleles.

As shown in Table 5, all the samples diagnosed as positive by ARMS have been detected as positive by LAMP. Out of 15 samples resulted negative by ARMS, 11 have been diagnosed as negative by LAMP and 4 as low positive. To exclude that the 4 discordant samples resulted mutated by LAMP were false positive and to confirm that the mutation diagnosis was due to an higher selectivity of the modified-LAMP method, we tested the samples using a third assay. The assay consists in PCR amplification of the JAK2 region of interest in presence of the PNA molecule complementary to the wild type target. The purpose is to enrich the mutated base, if present, by suppression of the wild type via PNA clamping. If the mutated region is enriched to a level of 20% of the sample, it can be detected by the direct-sequencing. The primers (GA231 forward and GA232 reverse) and the PNA are the same described above (paragraph "Example 4"). The amplification was performed in presence of 1x reaction buffer, 2.5mM MgCl₂, 200µM dNTPs, 500nM forward and reverse primers, 1.5M PNA and 0.025U Taq Gold in a final volume of 45µl. 5 µl of target 20ng/µl was added to the reaction mix. The resulting solution was incubated in a thermocycler, following a thermal program consisting in 10min at 95°C followed by 35 cycles of 30sec at 94°C, 40 sec at 62°C cycles, 30sec at 58°C and 30 sec at 72°C and finishing with 10min at 72°C for the final extension. The four discordant clinical samples, one no-target control sample and a positive and negative plasmid target were tested in duplicate. The resulting amplification products were separated on an agarose gel containing EtBr to visualize the amplification bands. The no-target control was not amplified. The negative control containing the wild type plasmid was slightly amplified, and a weak band was visible on the agarose gel. The positive control containing the mutated plasmid was strongly amplified presenting a strong band on the agarose gel. The clinical samples were amplified. The amplification products were consequently analyzed via automatic-sequencing. All the discordant clinical samples show a double peak in position 1849, corresponding to the Guanine (wild type) base and the Thymine (mutated) base. This result confirm that the four discordant samples have been correctly diagnosed mutated by LAMP, while they results false negative by ARMS.

**Table 5**

| sample | ARMs | LAMP self-annealed loop primer with PNA |
|---|---|---|
| PIGI | - | + (low) |
| PEVI | - | - |
| ACGI | + | + |
| BEMA | + | + |
| BILU | + | + |
| PAAN | - | + (low) |
| BOMA | - | - |
| OLIN | - | - |
| PALO | + | + |
| BOED | + | + |
| SAGE | + | + |
| BAGI | + | + |
| FEGI | + | + |
| BEAL | + | + |
| TALU | + | + |
| CAPI | + | + |
| SAGI | + | + |
| SAVGI | + | + |
| PECA | + | + |
| SCLU | - | - |
| BILU2 | - | + (low) |
| NAGI | - | - |
| MAST | - | - |
| COSA | - | - |
| GUAL | - | - |
| COCL | - | - |
| PEMG | - | - |
| SABA | - | + (low) |
| ANPI | - | - |

### EXAMPLE 6 - Fluorescent JAK2-modified-LAMP "Self-annealing Loop primer strategy"

### Reagents

JAK2 plasmids were synthesized by the supplier GeneArt (Regensburg, Germany) to contain the wild type or the mutant JAK2 sequence. In details:
- which Insert corresponds to the sequence 1689-1722 of MN_004972, including a G base at nucleotide1849, referred to as *"wt plasmid"*
- which Insert corresponds to the sequence 1689-1722 of MN_004972 cloned, including a T base at nucleotide1849, referred to as *"mut plasmid"*

Primers: synthesized by Eurofins MWG Operon, referred to as *"primers":*
- GA231 (F3) 5' GCATCTTTATTATGGCAGAGAG 3' (Seq Id No. 16)
- GA232 (B3) 5' TGCTCTGAGAAAGGCATTA 3' (Seq Id No. 17)
- GA233 (FIP)
   5' GCTGCTTCAAAGAAAGACTAAGGAAATGGACAACAGTCAAACAAC 3' (Seq Id No. 18)
- GA234 (BIP)
   5'GCTTTCTCACAAGCATTTGGTTTTAAATTAGCCTGTAGTTTTACTTACTCTC 3' (Seq Id No. 19)
   GA236 (LB) 5'TAMRA-TGTCTCCACTGGAGTATGTTTCTGTGGAGAC 3' (Seq Id No. 21). The underlined base corresponds to the mutated nucleotide at position 1849 of the JAK2 gene. The bold Tymine base at 5' end is not complementary to the target sequence. It has been added to separe the fluorophore from the Guanine base downstream, which has a quenching effect.
- GA235 (LF) 5'-5'GTCTCCACTGGAGTATGTGTCTGTGGAGAddC3'(Seq Id No. 20) the underlined base is the wild type nucleotide in position 1849 of the JAK2 gene. ddC stands for not-extensible dideoxy-citosine.
Reaction buffer: 100mM Tris HCl pH 8.8, 50mM KCl, 40mM MgSO4, 50mM (NH4)2SO4, 0.5% Tween, *"buffer 5x"*
dNTPs mix 25mM (Fermentas), *"dNTPs"*
Bst Large Fragment polymerase 8U/µl (New England Biolabs), *"Polymerase."*
Sterile apyrogen water (SALF Spa), *"ddw"*

### Procedure

### Sample preparation

Stock the primers in aliquots. It is better to store stock solutions at -20°C, while working dilutions should be stored at 4°C

Prepare reaction mix as follows: 0.2µM outer primers (F3 and B3), 1.6µM inner primers (FIP and BIP), 0.8µM fluorescent self-annealed loop primer specific for mutant JAK2 (LB), 0.8µM self annealed not-extensible loop primer for wild type JAK2 (LF), 1 x buffer solution, 1.4mM dNTPs mix, 8 U Bst Polymerase. Final volume of the reaction mix must be 4/5 of the total reaction volume (i.e. 20µl reaction mix + 5µl sample). Always keep reagents on ice. Prepare the mix for at least 23 samples, comprising 3 negative controls (100% wild type plasmid, 7e3 cps/µl), 18 positive control (3 samples 100% mutant plasmid, 3 samples 1% mutant plasmid diluted in wt plasmid, 3 samples 0.5% mutant plasmid diluted in wt plasmid, 3 samples 0.1% mutant plasmid diluted in wt plasmid, 3 samples 0.05% mutant plasmid diluted in wt plasmid, 3 samples 0.01% mutant plasmid diluted in wt plasmid (total amount of DNA 7e3 cps/µl), and one no target control.

**Table 6 - sample mix composition**

| Sample tube | 1-3 | 4-6 | 7-9 | 10-12 | 13-15 | 16-19 |
|---|---|---|---|---|---|---|
| Target to be add (5µl) | Wt plasmid 7e3 cps/µl | Mut plasmid 7e3 cps/µl | Mut plasmid 7e2 cps/µl | Mut plasmid 7e1 cps/µl | Mut plasmid 7e0 cps/µl | |
| GA231 100µM | 0.05 | 0.05 | 0.05 | 0.05 | 0.05 | 0.05 |
| GA232 100µM | 0.05 | 0.05 | 0.05 | 0.05 | 0.05 | 0.05 |
| GA233 100µM | 0.4 | 0.4 | 0.4 | 0.4 | 0.4 | 0.4 |
| GA234 100µM | 0.4 | 0.4 | 0.4 | 0.4 | 0.4 | 0.4 |
| GA235 100µM | 0.2 | 0.2 | 0.2 | 0.2 | 0.2 | 0.2 |
| GA236 100µM | 0.2 | 0.2 | 0.2 | 0.2 | 0.2 | 0.2 |
| Buffer LAMP 10x | 2.5 | 2.5 | 2.5 | 2.5 | 2.5 | 2.5 |
| Bst Polymerase 8U/µl | 1 | 1 | 1 | 1 | 1 | 1 |
| dNTPs 25mM | 1.4 | 1.4 | 1.4 | 1.4 | 1.4 | 1.4 |
| Ddw to 20 µl | 13.8 | 13.8 | 13.8 | 13.8 | 13.8 | 13.8 |
| | | | | | | |

Dispense 20µl of reaction mix in the strip. Keep the strips on ice. Always keep the reaction mix on ice from now on.

Prepare serial dilutions of the target (*"target dilutions"*) from shipped solution (wt plasmid and mut plasmid). Shipped solution is a 7*10¹⁰ copies/µl. Dilute initially the mutant plasmid to a 7*10⁴ copies/µl in Tris 10mM, then dilute serially the mutant plasmid in wt plasmid to obtain the following concentrations of mutant sequences in wild type background: 1%, 0.5%, 0.1%, 0.05%, 0.01% (total amount per tube, 7e3 cps/ul). Add 5µl of target dilutions to the strips, in triplicate. Add 5ul of the target dilutions starting from the less concentrated one to the most concentrated one. Close all the tubes.

### Reaction

The reaction follows the method scheme of Figure 4.

Program the real time instrument for incubation at constant temperature in order to obtain a constant reaction temperature of 65°C for 1 hour. Program the real time instrument in order to obtain a fluorescence reading per minute.

Put the strips in the instrument immediately before the beginning of the programs. Start the program.

### Data analysis

The fluorescent self-annealed loop primer in reaction produces a fluorescent signal once it is excited by an appropriated wavelenght-light emission. When the LAMP reaction proceeds, the fluorescent self-annealed loop primer is incorporated in the amplification products, being consequently annealed to a complementary nucleotide sequence. The fluorescent self-annealed loop primer is designed to be complementary to a sequence containing at least one Guanine nucleotide close to its 5' end. The Guanine base can absorbe the wavelengh emitted by the fluorophore (TAMRA in our case), causing a fluorescent signal quenching. The LAMP reaction can be consequently detected by analysis of the variation of fluorescence in terms of signal quenching, to find the threshold time relative to each analyzed sample. The threshold time is the minute at which the fluorescence signal in reaction reaches 50% of quenching. The threshold time reached by each samples is correlated with its Log of DNA copies/µl.

### Results

This approach consists of a selective mutant amplification based on a particular loop primer design resulting in selective hybridization of such loop primer to the dumb-bell formed from the mutant sequence (Figure 4). We designed a universal (mutant insensitive) set of primers comprising F3, B3, FIP and BIP to obtain a dumb-bell presenting the putative mutated nucleotide in the loop region comprised between B1 and B2. Other experiments were performed presenting the putative mutated nucleotide in the loop region into B2 or comprised between B2 and B1c, with no relevant differences. We included in the primer set a particular loop primer presenting a 8-bases sequence region at its 5' end complementary to its own sequence in 3' end.

Consequently, this special loop primer forms an intra-molecular hairpin structure in equilibrium with its open form at the reaction temperature (65°C).

When the mutated JAK2 sequence is present, this modified loop primer breaks its internal structure to anneal to the target, thanks to the thermodynamic equilibrium (Tm between primer and specific target = 65°C). The loop primer annealed to the specific mutated target is consequently extensible by the polymerase: the amplification can proceed.

When the wt sequence is present in the sample, the same loop primer (specific for the MUT JAK2 gene) presents a Tm with aspecific target ( 59°C) lower than the intra-molecular hairpin structure (65°C). This results in auto-sequestration of the modified loop primer that prefers to fold in the hairpin structure rather than to form a duplex with aspecific target, since the intramolecular forces are higher than the intermolecular ones. To limit the competition of the loop primer previously described for the wild type sequences likely to be present in large excess in the clinical sample, we added another modified loop primer characterized by a structure similar to the one previously described, but whit a sequence complementary to the JAK2 wild type sequence (with G base at position 1489).

The 3' end of this "competitor" loop primer is made not extensible by a modification (3' dideoxy). The task of this competitor is to "silence" the wt and allow the specific mutant primer to find its target.

When the "competitor" recognizes the specific wild type sequence, it breaks its intramolecular structure to anneal to the WT target, thanks to a higher affinity (Tm duplex wt target-wt modified loop primer= 67°C); The loop primer annealed to the wt target is not extensible, resulting in no amplification of the wt sequences. Since the reaction is conducted at constant temperature, the wt-loop primer will remain annealed to the wt sequences preventing aspecific annealing of the MUT loop primer.

Differently, the "competitor" presents a Tm with its aspecific (mutant) target ( 62°C) lower than the intra-molecular hairpin structure that it forms with itself (65°C). This results in auto-sequestration of the modified loop primer that prefers to fold in the hairpin structure rather than to form a duplex with the aspecific target, since the intramolecular forces are higher than the intermolecular ones.

To follow the reaction on a real-time instrument, we labeled the 5' end of the mutant modified loop primer with a FAM dye. To avoid the binding of the fluorophore to the guanine base present at the 5' end of the modified loop primer, which has a quenching effect, we added a Thymine base to the extremity of the probe. The modified-labeled primer, when present in solution, emits a fluorescent signal if excited by an appropriate-wavelength light. When the LAMP reaction starts and proceeds, the fluorescent self-annealed loop primer is incorporated in the amplification products, being consequently annealed to a complementary nucleotide sequence, containing several Guanine residues. The Guanine bases can absorb the wavelength emitted by the fluorophore, causing a fluorescent signal quenching visible in real time. The LAMP reaction can be consequently monitored throughout the analysis of the decreasing of fluorescence signal due to the "quenching effect" determined by LAMP product generation.

### References

1. Levine, R. L. et al. Activating mutation in the tyrosine kinase JAK2 in polycythemia vera, essential thrombocythemia, and myeloid metaplasia with myelofibrosis. Cancer Cell 7, 387-397 (2005).
2. James, C. et al. A unique clonal JAK2 mutation leading to constitutive signalling causes polycythaemia vera. Nature 434, 1144-1148 (2005).
3. Baxter, E. J. et al. Acquired mutation of the tyrosine kinase JAK2 in human myeloproliferative disorders. Lancet 365, 1054-1061 (2005).
4. Kralovics, R. et al. A gain of function mutation of JAK2 in myeloproliferative disorders. N. Engl. J. Med. 352, 1779-1790 (2005).
5. Nelson ME, Steensma DP: JAK2 V617F in myeloid disorders: what do we know now, and where are we headed? Leuk Lymphoma 2006, 47:177-194
6. James C, Ugo V, Le Couedic J-P, Staerk J, Delhommeau F, Lacout C, Garcon L, Raslova H, Berger R, Bennaceur-Griscelli A, Villeval JL, Constantinescu SN, Casadevall N, Vainchenker W: A unique clonal JAK2 mutation leading to constitutive signalling causes polycythaemia vera. Nature 2005, 434:1144-1148
7. De Keersmaecker K, Cools J: Chronic myeloproliferative disorders: a tyrosine kinase tale. Leukemia 2005, 20:200-205
8. Kaushansky K: On the molecular origins of the chronic myeloproliferative disorders: it all makes sense. Blood 2005, 105:4187-4190
9. Baxter EJ, Scott LM, Campbell PJ, East C, Fourouclas N, Swanton S, Vassiliou GS, Bench AJ, Boyd EM, Curtin N, Scott MA, Erber WN, Green AR: Acquired mutation of the tyrosine kinase JAK2 in human myeloproliferative disorders. The Lancet 2005, 365:1054-1061
10. Smith TA, Whelan J, Parry PJ: Detection of single-base mutations in a mixed population of cells: a comparison of SSCP and direct sequencing. Genet Anal Tech Appl 1992, 9:143-145
11. James C, Delhommeau F, Marzac C, Teyssandier I, Couedic JP, Giraudier S, Roy L, Saulnier P, Lacroix L, Maury S, Tulliez M, Vainchenker W, Ugo V, Casadevall N: Detection of JAK2 V617F as a irst intention diagnostic test for erythrocytosis. Leukemia 2006, 20:350-353
12. Newton CR, Graham A, Heptinstall LE, Powell SJ, Summers C, Kalsheker N, Smith JC, Markham AF: Analysis of any point mutation in DNA: the amplification refractory mutation system (ARMS). Nucleic Acids Res 1989, 17:2503-2516
13. Jones AV, Kreil S, Zoi K, Waghom K, Curtis C, Zhang L, Score J, Seear R, Chase AJ, Grand FH, White H, Zoi C, Loukopoulos D, Terpos E, Vervessou EC, Schultheis B, Emig M, Ernst T, Lengfelder E, Hehlmann R, Hochhaus A, Oscier D, Silver RT, Reiter A, Cross NC: Widespread occurrence of the JAK2 V617F mutation in chronic myeloproliferative disorders. Blood 2005, 106:2162-2168
14. James C, Delhommeau F, Marzac C, Teyssandier I, Couedic JP, Giraudier S, Roy L, Saulnier P, Lacroix L, Maury S, Tulliez M, Vainchenker W, Ugo V, Casadevall N: Detection of JAK2 V617F as a first intention diagnostic test for erythrocytosis. Leukemia 2006, 20:350-353
15. Baxter EJ, Scott LM, Campbell PJ, East C, Fourouclas N, Swanton S, Vassiliou GS, Bench AJ, Boyd EM, Curtin N, Scott MA, Erber WN, Green AR: Acquired mutation of the tyrosine kinase JAK2 in human myeloproliferative disorders. The Lancet 2005, 365:1054-1061
16. Antonioli E, Guglielmelli P, Pancrazzi A, Bogani C, Verrucci M, Ponziani V, Longo G, Bosi A, Vannucchi AM: Clinical implications of the JAK2 V617F mutation in essential thrombocythemia. Leukemia 2005, 19:1847-1849
17. Jelinek J, Oki Y, Gharibyan V, Bueso-Ramos C, Prchal JT, Verstovsek S, Beran M, Estey E, Kantarjian HM, Issa JP: JAK2 mutation 1849G_T is rare in acute leukemias but can be found in CMML, Philadelphia chromosome-negative CML, and megakaryocytic leukemia. Blood 2005, 106:3370-3373
18. Jones AV: Widespread occurence of the JAK2 V617F mutation in chronic myeloproliferative disorders. Blood 2005, 106: 2162-2168.
19. Steensma D: JAK2 V617F in myeloid disorders: molecular diagnostic techniques and their clinical utility. Journal of Molecular Diagnostics 2006, 8:397-411.
20. Iwasaki M. et al. Genome Letters 2003, 2:119-126.
21. Fukuta S. et al. J Appl Genet 2006, 47:303-308.
22. Ikeda S. et al. Pathol. Intern. 2007, 57: 594-599.

## Claims

1. Method for detecting the presence of a point mutation of a target nucleic acid molecule in a background of nucleic acid wild type molecules, comprising the steps of:
a. obtaining a nucleic acid sample;
b. contacting said nucleic acid sample, in appropriate reaction conditions, with a solution comprising a mixture of oligonucleotides and a DNA polymerase with strand displacement activity under hybridization conditions, wherein said mixture of oligonucleotides consists of primers suitable for a loop mediated isothermal amplification of the region of the nucleic acid molecule putatively including the point mutation, said primers comprising :
i. two outer primers F3 and B3 ;
ii. two inner primers FIP and BIP, where FIP includes two oligonucleotide sequences, F2 and F1c, and BIP includes two oligonucleotide sequences, B2 and B1c, where said inner primers FIP and BIP are able to recognize and hybridize to two different and opposite regions, F2c and B2c respectively, of the target nucleic acid molecule, where either the BIP primer is designed to hybridize downstream of the point mutation, or the FIP primer is designed to hybridize upstream of the point mutation, and in the case that the BIP primer is designed to hybridize downstream of the point mutation, then the FIP primer is designed to hybridize to the target sequence such that the point mutation is located in or downstream of the F2c sequence and upstream of F1 c, or in the case that the FIP primer is designed to hybridize upstream of the point mutation, then the BIP primer is designed to hybridize to the target sequence such that the point mutation is located in or upstream of the B2c sequence and downstream of B1c;
iii. a self-annealed extensible primer LB or LF respectively comprising: - a central loop sequence able to selectively recognize and hybridize to the region comprising the putative point mutation of the nucleic acid molecule only if the point mutation is present, - a 5' end sequence and - a 3' end sequence, said 5' end and said 3' end sequences being complementary to each other to form a stem, so that said central loop sequence has a higher hybridization affinity to the region comprising the putative point mutation of the nucleic acid molecule than the hybridization affinity of the 5' end sequence to the 3' and sequence, so that it results in annealing and amplification of the region comprising the putative point mutation of the nucleic add molecule;
iv. a non extensible moiety able to selectively recognize and hybridize to the WT sequence of nucleic acid molecule;
c. incubating the resulting mixture at a constant temperature;
d. detecting a signal indicative of amplification of the nucleotide molecule comprising the point mutation.

2. The method of claim 1 wherein the point mutation is located in the region between F2 and F1 c or B2 and B1c.

3. The method of claim 1 wherein the point mutation is located in the region probed by B2 or F2.

4. The method of any of the previous claims wherein the sequence at the 5' end and the sequence at the 3' end of said self-annealed extensible primer is of at least 3 nucleotides.

5. The method of any of the previous claims wherein the non extensible moiety is a peptide nucleic acid (PNA).

6. The method according to claim 5 where said peptide nucleic acid (PNA) has at least 10 nucleotides.

7. The method according to claim 6 where said peptide nucleic acid (PNA) comprises a sequence of bases capable of hybridizing with the region including the putative point mutation resulting in double strand structures having respectively a melting temperature (Tm) = X with the wild type sequence and melting temperature (Tm) = Y with the mutant sequence, where Y< Incubation Temperature ≤ X and X is at least 5°C higher than Y.

8. The method according to claims 1 to 4 wherein the non extensible moiety is a self-annealed non extensible primer, comprising a central loop sequence able to selectively recognize and hybrizide to the region comprising the wild type sequence of the nucleic acid molecule, a 5' end sequence and a 3' end sequence, said 5' end and said 3' end sequences being complementary to each other to form a stem, so that said central loop sequence has a higher hybridization affinity to the region comprising the wild type sequence of the nucleic acid molecule than the hybridization affinity of the 5' end sequence to the 3' and sequence, so that it results in annealing and blocking of the wt sequence.

9. The method according to any of previous claims wherein said DNA polymerase with strand displacement activity is the Bst large fragment polymerase.

10. The method according to any of the previous claims 1 to 8 wherein said DNA polymerase with strand displacement activity is one of or a combination of: Bca (exo-), Vent, Vent (exo-), Deep Vent, Deep Vent (exo-), Φ29 phage, MS-2 phage, Z-Taq, KOD, Klenow fragment.

11. The method according to any of the previous claims wherein the constant temperature is comprised between 62 and 67 °C.

12. The method according to any of the previous claims wherein the signal indicative of amplification of the nucleotide molecule comprising the point mutation is detected by turbidimetry.

13. The method according to any of the previous claims 1 to 11 wherein the signal indicative of amplification of the nucleotide molecule comprising the point mutation is detected by fluorescence.

14. The method according to any of the previous claims wherein the nucleic acid molecule comprises the region of the human JAK2 gene (GenBank accession no. NM_004972 illustrated in Figure 11a,b,c, putatively having the point mutation, guanine-to-thymidine substitution at base 1849 (G1849T).

15. The method according to claim 14 wherein F2 and F1c are in position 1730-1750 and 1770-1795 respectively of the NM_004972 gene sequence; B2 is in position 1862-1884 of the NM_004972 gene sequence and B1c is in position 1810-1840 of the NM_004972 gene sequence illustrated in Figure 11a,b,c.

16. The method according to claim 15 wherein the primers have the following sequences:
F3 5'- GCATCTTTATTATGGCAGAGAG- 3' (Seq Id No.1);
B3 5'-TGCTCTGAGAAAGGCATTA- 3' (Seq Id No. 2);
FIP 5'-GCTGCTTCAAAGAAAGACTAAGGAAATGGACAACAGTCAAACAAC -3' (Seq Id No. 3);
BIP 5'-GCTTTCTCACAAGCATTTGGTTTTAAATTAGCCTGTAGTTTTACTTACTCTC-3' (Seq Id No. 4).

17. The method according to claims 15 or 16 wherein said non extensible moiety is a PNA molecule.

18. The method according to claim 16 wherein said PNA molecule has the structure: ^{NH2}GAGTATGTGTCTGTGGA^{CONH2}.

## Patentansprüche

1. Verfahren zur Detektion des Vorhandenseins einer Punktmutation eines Zielnukleinsäuremoleküls in einem Hintergrund von Wildtyp-Nukleinsäuremolekülen, umfassend die folgenden Schritte:
a. Gewinnen einer Nukleinsäureprobe;
b. Kontaktieren der Nukleinsäureprobe unter geeigneten Reaktionsbedingungen mit einer Lösung, umfassend eine Mischung aus Oligonukleotiden und einer DNA-Polymerase mit Strangverdrängungsaktivität unter Hybridisierungsbedingungen, wobei die Mischung aus Oligonukleotiden aus Primern besteht, die für eine Schleifen-vermittelte isothermische Amplifikation der Region des Nukleinsäuremoleküls geeignet ist, die mutmaßlich die Punktmutation enthält, wobei die Primer folgendes umfassen:
i. zwei äußere Primer F3 und B3;
ii. zwei innere Primer FIP und BIP, wobei FIP zwei Oligonukleotidsequenzen, F2 und F1c, umfasst und BIP zwei Oligonukleotidsequenzen, B2 und B 1 c, umfasst, wobei die inneren Primer FIP und BIP in der Lage sind, zwei verschiedene und entgegengesetzte Regionen, F2c bzw. B2c, des Zielnukleinsäuremoleküls zu erkennen und damit zu hybridisieren, wobei entweder der BIP-Primer ausgelegt ist, stromabwärts der Punktmutation zu hybridisieren oder der FIP-Primer ausgelegt ist, stromaufwärts der Punktmutation zu hybridisieren, und in dem Fall, dass der BIP-Primer ausgelegt ist, stromabwärts der Punktmutation zu hybridisieren, der FIP-Primer dann ausgelegt ist, mit der Zielsequenz zu hybridisieren, so dass sich die Punktmutation in oder stromabwärts der F2c-Sequenz und stromaufwärts von F1c befindet, oder in dem Fall, dass der FIP-Primer ausgelegt ist, stromaufwärts der Punktmutation zu hybridisieren, der BIP-Primer dann ausgelegt ist, mit der Zielsequenz zu hybridisieren, so dass sich die Punktmutation in oder stromaufwärts der B2c-Sequenz und stromabwärts von B1c befindet;
iii. einen selbst-bindenden, verlängerbarer Primer LB bzw. LF, umfassend:
- eine zentrale Schleifensequenz, die nur in der Lage ist, selektiv die Region, die mutmaßlich die Punktmutation des Nukleinsäuremoleküls umfasst, zu erkennen und damit zu hybridisieren, wenn die Punktmutation vorhanden ist,
- eine 5 `-Endsequenz - und eine 3'-Endsequenz, wobei die 5'-Endsequenz und 3'-Endsequenz komplementär zueinander sind, um einen Stamm zu bilden, so dass die zentrale Schleifensequenz eine höhere Hybridisierungsaffinität zur Region, welche die mutmaßliche Punktmutation des Nukleinsäurennukleotids umfasst, aufweist als die Hybridisierungsaffinität der 5'-Endsequenz zur 3'-Endsequenz, so dass es in einer Bindung und Amplifikation der Region resultiert, welche die mutmaßliche Punktmutation des Nukleinsäuremoleküls umfasst;
iv. einen nicht verlängerbaren Teil, der in der Lage ist, selektiv die WT-Sequenz des Nukleinsäuremoleküls zu erkennen und damit zu hybridisieren;
c. Inkubieren des resultierenden Gemisches bei einer konstanten Temperatur;
d. Detektieren eines Signals, das eine Amplifikation des Nukleotidmoleküls anzeigt, das die Punktmutation umfasst.

2. Verfahren nach Anspruch 1, wobei sich die Punktmutation in der Region zwischen F2 und F1c oder B2 und B1c befindet.

3. Verfahren nach Anspruch 1, wobei sich die Punktmutation in der Region befindet, die durch B2 oder F2 sondiert wird.

4. Verfahren nach einem der vorstehenden Ansprüche, wobei die Sequenz am 5'-wende und die Sequenz am 3`-Ende des selbst-bindenden, verlängerbaren Primers zumindest aus 3 Nukleotiden besteht.

5. Verfahren nach einem der vorstehenden Ansprüche, wobei der nicht verlängerbare Teil eine Peptidnukleinsäure (PNS) ist.

6. Verfahren nach Anspruch 5, wobei die Peptidnukleinsäure (PNS) zumindest 10 Nukleotide aufweist.

7. Verfahren nach Anspruch 6, wobei die Peptidnukleinsäure (PNS) eine Sequenz aus Basen umfasst, die in der Lage ist, mit der Region, welche die mutmaßliche Punktmutation enthält, zu hybridisieren, was in Doppelstrangstrukturen resultiert, die jeweils eine Schmelztemperatur (Tm) = X mit der Wildtyp-Sequenz und eine Schmelztemperature (Tm) = Y mit der mutierten Sequenz aufweisen, wobei Y < Inkubationstemperatur ≤ X und X zumindest um 5°C höher als Y ist.

8. Verfahren nach den Ansprüchen 1 bis 4, wobei der nicht verlängerbare Teil ein selbstbindender, nicht verlängerbarer Primer ist, umfassend eine zentrale Schleifensequenz, die in der Lage ist, selektiv die Region, welche die Wildtyp-Sequenz des Nukleinsäuremoleküls umfasst, zu erkennen und damit zu hybridisieren, eine 5'-Endsequenz und eine 3'-Endsequenz, wobei die 5'-Endsequenz und die 3'-Endsequenz zueinander komplementär sind, um einen Stamm zu bilden, so dass die zentrale Schleifensequenz eine höhere Hybridisierungsaffinität zur Region, welche die Wildtyp-Sequenz des Nukleinsäuremoleküls umfasst, aufweist als die Hybridisierungsaffinität der 5'-Endsequenz zur 3'-Endsequenz, so dass es in einer Bindung und Blockierung der wt-Sequenz resultiert.

9. Verfahren nach einem der vorstehenden Ansprüche, wobei die DNA-Polymerase mit Strangverdrängungsaktivität die Bst-Großfragment-Polymerase ist.

10. Verfahren nach einem der vorstehenden Ansprüche 1 bis 8, wobei die DNA-Polymerase mit Strangverdrängungsaktivität eine oder eine Kombination von Folgenden ist: Bca (exo-), Vent, Vent (exo), Deep Vent, Deep Vent (exo-), Φ 29 Phage, MS-2 Phage, Z-Taq, KOD, Klenow-Fragment.

11. Verfahren nach einem der vorstehenden Ansprüche, wobei die konstante Temperatur zwischen 62 und 67°C liegt.

12. Verfahren nach einem der vorstehenden Ansprüche, wobei das Signal, das die Amplifikation des Nukleotidmoleküls, das die Punktmutation umfasst, anzeigt, mittels Turbidimetrie detektiert wird.

13. Verfahren nach einem der vorstehenden Ansprüche 1 bis 11, wobei das Signal, das die Amplifikation des Nukleotidmoleküls, das die Punktmutation umfasst, anzeigt, mittels Fluoreszenz detektiert wird.

14. Verfahren nach einem der vorstehenden Ansprüche, wobei das Nukleinsäuremolekül die Region des humanen JAK2-Gens (GenBank Zugriffsnummer NM_004972, in Figur 11a, b, c veranschaulicht) umfasst, die mutmaßlich die Punktmutation, Guanin-zu-Thymidin-Substitution an der Base 1849 (G1849T), aufweist.

15. Verfahren nach Anspruch 14, wobei F2 und F1c sich an Position 1730-1750 bzw. 1770-1795 der NM_004972-Gensequenz befinden; B2 sich an Position 1862-1884 der NM_004972-Gensequenz befindet und B1c sich an Position 1810-1840 der NM_004972-Gensequenz befindet, veranschaulicht in Figur 11a, b, c.

16. Verfahren nach Anspruch 15, wobei die Primer die folgenden Sequenzen aufweisen:
F3 5'- GCATCTTTATTATGGCAGAGAG- 3' (Seq Id No. 1);
B3 5'-TGCTCTGAGAAAGGCATTA- 3'(Seq Id No. 2);
FIP 5'-GCTGCTTCAAAGAAAGACTAAGGAAATGGACAACAGTCAAACAAC -3' (Seq Id No. 3);
BIP 5'-GCTTTCTCACAAGCATTTGGTTTTAAATTAGCCTGTAGTTTTACTTACTCTC-3' (Seq Id No. 4).

17. Verfahren nach den Ansprüchen 15 oder 16, wobei der nicht verlängerbare Teil ein PNS-Molekül ist.

18. Verfahren nach Anspruch 16, wobei das PNS-Molekül die folgende Struktur aufweist:
^{NH2}GAGTATGTGTCTGTGGA^{CONH2}.

## Revendications

1. Procédé de détection de la présence d'une mutation ponctuelle dans une molécule d'acide nucléique cible dans un contexte de molécules d'acide nucléique de type sauvage, qui comprend les étapes suivantes :
a. obtention d'un échantillon d'acide nucléique ;
b. mise en contact dudit échantillon d'acide nucléique, dans des conditions de réaction adaptées, avec une solution comprenant un mélange d'oligonucléotides et une ADN polymérase ayant une activité de déplacement de brin dans des conditions d'hybridation, dans lequel ledit mélange d'oligonucléotides est constitué par des amorces adaptées pour une amplification isothermique médiée par boucle de la région de la molécule d'acide nucléique comprenant supposément la mutation ponctuelle, lesdites amorces comprenant:
i. deux amorces externes F3 et B3 ;
ii. deux amorces internes FIP et BIP, FIP comprenant deux séquences d'oligonucléotides, F2 et F1 c, et BIP comprenant deux séquences d'oligonucléotides, B2 et B1 c, lesdites amorces internes FIP et BIP étant capables de reconnaître deux régions différentes et opposées et de s'y hybrider, F2c et B2c respectivement, de la molécule d'acide nucléique cible, où soit l'amorce BIP est conçue pour s'hybrider en aval de la mutation ponctuelle, soit l'amorce FIP est conçue pour s'hybrider en amont de la mutation ponctuelle, et dans le cas où l'amorce BIP est conçue pour s'hybrider en aval de la mutation ponctuelle, alors l'amorce FIP est conçue pour s'hybrider à la séquence cible de telle sorte que la mutation ponctuelle se trouve dans la séquence F2c ou en aval de celle-ci et en amont de F1c, ou dans le cas où l'amorce FIP est conçue pour s'hybrider en amont de la mutation ponctuelle, alors l'amorce BIP est conçue pour s'hybrider à la séquence cible de telle sorte que la mutation ponctuelle se trouve dans la séquence B2c ou en amont de celle-ci et en aval de B1c ;
iii. une amorce LB ou LF extensible et auto-hybridée comprenant respectivement :
- une séquence de boucle centrale capable de sélectivement reconnaître la région comprenant la mutation ponctuelle supposée de la molécule d'acide nucléique uniquement si la mutation ponctuelle est présente et de s'hybrider à ladite région, - une séquence d'extrémité 5' et - une séquence d'extrémité 3', ladite séquence d'extrémité 5' et ladite séquence d'extrémité 3' étant complémentaires l'une de l'autre pour former une tige, de telle sorte que ladite séquence de boucle centrale présente une affinité d'hybridation envers la région comprenant la mutation ponctuelle supposée de la molécule d'acide nucléique supérieure à l'affinité d'hybridation de la séquence d'extrémité 5' à la séquence d'extrémité 3', résultant ainsi en l'hybridation et en l'amplification de la région comprenant la mutation ponctuelle supposée de la molécule d'acide nucléique ;
iv. une fraction non extensible capable de sélectivement reconnaître la séquence de type sauvage de la molécule d'acide nucléique et de s'y hybrider ;
c. incubation du mélange obtenu à une température constante ;
d. détection d'un signal indiquant l'amplification de la molécule nucléotidique comprenant la mutation ponctuelle.

2. Procédé selon la revendication 1, dans lequel la mutation ponctuelle se trouve dans la région entre F2 et F1 c ou B2 et B1c.

3. Procédé selon la revendication 1, dans lequel la mutation ponctuelle se trouve dans la région sondée par B2 ou F2.

4. Procédé selon l'une quelconque des revendications précédentes, dans lequel la séquence au niveau de l'extrémité 5' et la séquence au niveau de l'extrémité 3' de ladite amorce extensible auto-hybridée sont constituées d'au moins 3 nucléotides.

5. Procédé selon l'une quelconque des revendications précédentes, dans lequel la fraction non extensible est un acide nucléique peptidique (ANP).

6. Procédé selon la revendication 5, dans lequel ledit acide nucléique peptidique (ANP) comprend au moins 10 nucléotides.

7. Procédé selon la revendication 6, dans lequel ledit acide nucléique peptidique (ANP) comprend une séquence de bases capable de s'hybrider avec la région comprenant la mutation ponctuelle supposée résultant en des structures à double brin ayant respectivement une température de fusion (Tm) = X avec la séquence de type sauvage et une température de fusion (Tm) = Y avec la séquence mutante, où Y < température d'incubation ≤ X et X est supérieur d'au moins 5°C à Y.

8. Procédé selon les revendications 1 à 4, dans lequel la fraction non extensible est une amorce non extensible auto-hybridée, qui comprend une séquence de boucle centrale capable de sélectivement reconnaître la région comprenant la séquence de type sauvage de la molécule d'acide nucléique et de s'hybrider à ladite région, une séquence d'extrémité 5' et une séquence d'extrémité 3', ladite séquence d'extrémité 5' et ladite séquence d'extrémité 3' étant complémentaires l'une de l'autre pour former une tige, de telle sorte que ladite séquence de boucle centrale possède une affinité d'hybridation pour la région comprenant la séquence de type sauvage de la molécule d'acide nucléique supérieure à l'affinité d'hybridation de la séquence d'extrémité 5' à la séquence d'extrémité 3', résultant ainsi en une hybridation et un blocage de la séquence de type sauvage.

9. Procédé selon l'une quelconque des revendications précédentes, dans lequel ladite ADN polymérase ayant une activité de déplacement de brin est la polymérase du grand fragment de Bst.

10. Procédé selon l'une quelconque des revendications précédentes 1 à 8, dans lequel ladite ADN polymérase ayant une activité de déplacement de brin est l'une de ou une combinaison de : Bca (exo-), Vent, Vent (exo-), Deep Vent, Deep Vent (exo-), phage φ29, phage MS-2, Z-Taq, KOD, fragment de Klenow.

11. Procédé selon l'une quelconque des revendications précédentes, dans lequel la température constante est comprise entre 62 et 67°C.

12. Procédé selon l'une quelconque des revendications précédentes, dans lequel le signal indiquant l'amplification de la molécule nucléotidique comprenant la mutation ponctuelle est détecté par turbidimétrie.

13. Procédé selon l'une quelconque des revendications précédentes 1 à 11, dans lequel le signal indiquant l'amplification de la molécule nucléotidique comprenant la mutation ponctuelle est détecté par fluorescence.

14. Procédé selon l'une quelconque des revendications précédentes, dans lequel la molécule d'acide nucléique comprend la région du gène JAK2 humain (n° d'accès GenBank : NM_004972, illustré sur la figure 11a, b, c), ayant supposément la mutation ponctuelle, une substitution guanine à thymidine au niveau de la base 1849 (G1849T).

15. Procédé selon la revendication 14, dans lequel F2 et F1 c se trouvent aux positions 1730 à 1750 et 1770 à 1795, respectivement, de la séquence génique NM_004972 ; B2 se trouve aux positions 1862 à 1884 de la séquence génique NM_004972 et B1c se trouve aux positions 1810 à 1840 de la séquence génique NM_004972 illustrée sur la figure 11a, b, c.

16. Procédé selon la revendication 15, dans lequel les amorces ont les séquences suivantes :
F3 5'-GCATCTTTATTATGGCAGAGAG-3' (SEQ ID NO: 1) ;
B3 5'-TGCTCTGAGAAAGGCATTA- 3' (SEQ ID NO : 2) ;
FIP 5'-GCTGCTTCAAAGAAAGACTAAGGAAATGGACAACAGTCAAACAAC -3' (SEQ ID NO:3);
BIP 5'-GCTTTCTCACAAGCATTTGGTTTTAAATTAGCCTGTAGTTTTACTTACTCTC-3' (SEQ ID NO : 4).

17. Procédé selon les revendications 15 ou 16, dans lequel ladite fraction non extensible est une molécule d'ANP.

18. Procédé selon la revendication 16, dans lequel ladite molécule d'ANP a la structure : ^{NH2}GAGTATGTGTCTGTGGA^{CONH2}_{.}


### SEQUENCE LISTING

<110> DIASORIN SpA
<120> HIGHLY SENSITIVE RAPID ISOTHERMAL METHOD FOR THE DETECTION OF POINT MUTATIONS AND SNPs
<130> BE106814
<160> 22
<170> PatentIn version 3.5
<210> 1
   <211> 22
   <212> DNA
   <213> Artificial Sequence
<220>
   <223> synthetic oligonucleotide
<400> 1
   gcatctttat tatggcagag ag 22
<210> 2
   <211> 19
   <212> DNA
   <213> Artificial Sequence
<220>
   <223> synthetic oligonucleotide
<400> 2
   tgctctgaga aaggcatta 19
<210> 3
   <211> 45
   <212> DNA
   <213> Artificial Sequence
<220>
   <223> synthetic oligonucleotide
<400> 3
   gctgcttcaa agaaagacta aggaaatgga caacagtcaa acaac 45
<210> 4
   <211> 52
   <212> DNA
   <213> Artificial Sequence
<220>
   <223> synthetic oligonucleotide
<400> 4
   gctttctcac aagcatttgg ttttaaatta gcctgtagtt ttacttactc tc 52
<210> 5
   <211> 23
   <212> DNA
   <213> Artificial Sequence
<220>
   <223> synthetic oligonucleotide
<400> 5
   gtcaaacaac aattctttgt act 23
<210> 6
   <211> 19
   <212> DNA
   <213> Artificial sequence
<220>
   <223> synthetic oligonucleotide
<400> 6
   agctgtgatc ctgaaactg 19
<210> 7
   <211> 52
   <212> DNA
   <213> Artificial Sequence
<220>
   <223> synthetic oligonucleotide
<400> 7
   aatatactcc ataatttaaa accaaatgct ttctttcttt gaagcagcaa gt 52
<210> 8
   <211> 53
   <212> DNA
   <213> Artificial sequence
<220>
   <223> synthetic oligonucleotide
<400> 8
   ttttgtggag acgagagtaa gtaaaactac ataaacaaaa acagatgctc tga 53
<210> 9
   <211> 21
   <212> DNA
   <213> Artificial Sequence
<220>
   <223> synthetic oligonucleotide
<400> 9
   gtgagaaagc ttgctcatca t 21
<210> 10
   <211> 19
   <212> DNA
   <213> Artificial Sequence
<220>
   <223> synthetic oligonucleotide
<400> 10
   aggctttcta atgcctttc 19
<210> 11
   <211> 25
   <212> DNA
   <213> Artificial sequence
<220>
   <223> synthetic oligonucleotide
<400> 11
   tctatagtca tgctgaaagt aggag 25
<210> 12
   <211> 22
   <212> DNA
   <213> Artificial Sequence
<220>
   <223> synthetic oligonucleotide
<400> 12
   aaggcattag aaagcctgta gt 22
<210> 13
   <211> 55
   <212> DNA
   <213> Artificial Sequence
<220>
   <223> synthetic oligonucleotide
<400> 13
   acaaagaatt gttgtttgac tgttgtccat tgcatcttta ttatggcaga gagaa 55
<210> 14
   <211> 52
   <212> DNA
   <213> Artificial Sequence
<220>
   <223> synthetic oligonucleotide
<400> 14
   agtctttctt tgaagcagca agtatgatgt tacttactct cgtctccaca ga 52
<210> 15
   <211> 30
   <212> DNA
   <213> Artificial Sequence
<220>
   <223> synthetic oligonucleotide
<400> 15
   agcatttggt tttaaattat ggagtaggtt 30
<210> 16
   <211> 22
   <212> DNA
   <213> Artificial Sequence
<220>
   <223> synthetic oligonucleotide
<400> 16
   gcatctttat tatggcagag ag 22
<210> 17
   <211> 19
   <212> DNA
   <213> Artificial Sequence
<220>
   <223> synthetic oligonucleotide
<400> 17
   tgctctgaga aaggcatta 19
<210> 18
   <211> 45
   <212> DNA
   <213> Artificial Sequence
<220>
   <223> synthetic oligonucleotide
<400> 18
   gctgcttcaa agaaagacta aggaaatgga caacagtcaa acaac 45
<210> 19
   <211> 52
   <212> DNA
   <213> Artificial Sequence
<220>
   <223> synthetic oligonucleotide
<400> 19
   gctttctcac aagcatttgg ttttaaatta gcctgtagtt ttacttactc tc 52
<210> 20
   <211> 30
   <212> DNA
   <213> Artificial Sequence
<220>
   <223> synthetic oligonucleotide
<220>
   <221> misc_feature
   <222> (30)..(30)
   <223> not-extensible dideoxy-cytosine
<400> 20
   gtctccactg gagtatgtgt ctgtggagac 30
<210> 21
   <211> 30
   <212> DNA
   <213> Artificial Sequence
<220>
   <223> synthetic oligonucleotide
<400> 21
   gtctccactg gagtatgttt ctgtggagac 30
<210> 22
   <211> 30
   <212> DNA
   <213> Artificial Sequence
<220>
   <223> synthetic oligonucleotide
<400> 22
   gtctccactg gagtatgttt ctgtggagac 30